# EUROPEAN PATENT APPLICATION

(11) **EP 4 748 454 A1**
(43) Date of publication of application: **27.05.2026**
(21) Application number: 24214554.8
(22) Date of filing: 21.11.2024
(51) Int. Cl.: A61Q 5/10, A61Q 5/00, A61K 8/34, A61K 8/37, A61K 8/97

(54) **ANTI-GREY-HAIR COMPOSITION**

(71) Applicant: Wella Germany GmbH, 64295 Darmstadt (DE)
(72) Inventor: Nickel, Anne, Darmstadt (DE); Riemann, Ingo, Darmstadt (DE)
(74) Representative: Zimmermann & Partner Patentanwälte mbB

(57) **Abstract**

Compositions comprising an Avena strigosa extract, an Ononis spinosa extract, and 2-O-feruloyl-L-malic acid are suitable for reducing or preventing the greying of hair.

## Description

### FIELD OF THE INVENTION

The present invention relates to a composition for reducing or preventing the greying of hair, or for at least partially reverting the color of grey hair back to its original color. The composition comprises as active ingredients 2-O-feruloyl-L-malic acid, an extract of Avena strigosa, and an extract of Ononis spinosa. Optionally, the active ingredient 2-O-feruloyl-L-malic acid may be present in the form of an extract of Nasturtium officinale. The composition optionally may contain one or more additional active ingredients such as for example sandal pentanol (3-methyl-5-(2,2,3-trimethylcyclopent-3-en-1-yl)pentan-2-ol), caffeine and/or one or more saturated or unsaturated C10-C18-fatty acids, or salts thereof. In an aspect, the present invention relates to a composition, and the use of said composition for for reducing or preventing the greying of hair and improving scalp health.

### BACKGROUND OF THE INVENTION

The growth of human hair occurs in a known manner according to a natural growth rhythm including a growth phase (anagen), a transition phase (catagen), a resting phase (telogen) and a release phase (exogen). A normal natural hair cycle begins when a hair bottom end is ejected or cast away in exogen phase or prior to beginning of a new anagen phase. During the anagen phase the cells in the root of the hair are dividing rapidly so that new hair is formed, with a typical growth rate for hair on the scalp of approximately 0.3 to 0.4 millimeters per day. Anagen phase lasts about 2-6 years. Following the anagen phase, the catagen phase denotes a short transition phase wherein hair growth is stopped and the cells that produce hair growth are cut off from blood supply. Catagen phase lasts approximately ten days. During telogen phase, which lasts about 100 days, the individual hairs are still retained in the follicles but are no longer actively growing. Finally, at exogen phase the individual hairs are ejected or released. Exogen phase lasts approximately 2-5 months.

During normal hair growth, 80 to 100 hairs fall out from the scalp per day. This number is an approximate value, which can occur over a shorter time and also can be greater or smaller, without causing fear regarding natural hair growth.

Any loss of hair volume over the time is generally perceived as "thinning of hair", but such a volume loss may be caused by different phenomena. One obvious reason for perceived thinner hair is hair loss, i.e. the number or areal density of hair follicles producing hair decreases. Another reason for perceived thinner hair are alterations in the growth cycle. For example, the percentage of hairs in anagen phase and the duration of the anagen phase may diminish, resulting primarily in shorter hairs but perceived as an overall loss of hair volume. Another reason for perceived thinner hair is an alteration of the diameter or cross-section of the hair, i.e. a thinning of the individual hairs.

The color of hair results from pigments named melanins. Melanins are produced by specialized cells - the so-called melanocytes, which are located in the hair follicles. The are two maintypes of melanins - eumelanin and pheomelanin. Eumelanin is responsible for black and brown hair, while pheomelanin is responsible for red and blonde hair. The exact color of hair is determined by the specific combination of melanins and their concentration, which in turn is the result of one's genetic predisposition.

Greying of hair is part of the natural ageing process, which affects most adults with increasing age. Factors that have an impact on the greying of hair include a genetic influence, environmental factors, exposure to UV radiation, smoking, (mal)nutrition, and other factors including for example stress. The onset age of greying varies greatly. Although it is not uncommon for individuals that the hair starts to turn grey in their 20's, most people get their first grey hairs in the 30's and 40's. On the other hand, there is still a significant portion of individuals having few grey hairs at the age of 50 or even 60.

EP0116439A2 discloses a hair tonic composition, comprising as an effective ingredient at least one of specified C10-C20 fatty acid, or a salt thereof. The hair tonic composition prevents the generation of dandruff in hair and itching in the scalp, and is said to accelerate the growth of hair.

US2002/0034485A1 discloses a hair tonic preventing or at least reducing hair loss, which hair tonic includes at least one saturated and unsaturated C10-C18 fatty acid, or a salt thereof, biotin and/or caffeine.

US2019/0216696A1 discloses the use of specified derivates of sandal pentanol for promoting hair growth and/or inhibiting or delaying hair loss in the human scalp.

WO2020/069522A1 discloses a cosmetic formulation comprising an effective amount of sandal pentanol or analogs thereof, and minoxidil or rosemary oil or emu oil or a combination thereof for the treatment and regrowth of mammalian hair loss.

US2021/0259948A1 discloses a cosmetic composition having an anti- graying effect on hair, which composition comprises as a cosmetic active ingredient at least one extract of Avena strigosa, and at least one extract of Ononis spinosa.

WO2021/177430A1 discloses a composition for promoting hair growth and/or preventing grey hair, which composition comprises 2-O-feruloyl-L-malic acid.

A need exists for care products suitable for for reducing or even preventing the greying of hair. Even more, there is a need for care products suitable for reverting the color of grey hair back to its original color. Such care products should have no negative impact on the health generally, and preferably improve scalp health. Other sought-after effects of such care products include an improvement of the hair structure, an improved volume of hair - contrasting any preceived "thinning of hair", and an improvement of hair growth or reduction of hair loss.

### SUMMARY OF THE INVENTION

According to the present invention, it has now surprisingly been found out that a composition comprising a combination of a plant extract from Avena strigosa (black oat), a plant extract from Ononis spinosa (spiny restharrow), and 2-O-feruloyl-L-malic acid, is suitable for reducing or preventing the greying of hair.

Subject matter of the present invention is a composition comprising an Avena strigosa extract, an Ononis spinosa extract, and 2-O-feruloyl-L-malic acid. The active ingredient 2-O-feruloyl-L-malic acid optionally may be present in the form of a plant extract from Nasturtium officinale (watercress). Typically, the composition also comprises a solvent or solvent-water system.

The composition optionally may comprise additional active ingredients, such as for example sandal pentanol, caffeine, one or more saturated and unsaturated C10-C18 fatty acids, or a combination thereof.

The composition optionally may further comprise one or more components that provide for a beneficial effect (denoted herein as "supportive components"), one or more oil components, one or more mineral components, and one or more cosmetically acceptable additives.

According to embodiments, the composition is a ready-to-use scalp-and-hair treatment product selected from hair tonic, hair lotion, hair conditioner, hair cream, hair shampoo, hair rinse, hair liquid, hair oil, hair pomade, hair foam. Typically, the composition is a leave-on product. For example, the composition may be a hair tonic comprising a water-alcohol-based solvent system.

According to a further aspect, subject matter of the present invention is a method of treating hair. The method comprises applying a composition or scalp-and-hair treatment product according to the present invention to scalp. The method according to the present invention aims at reducing or preventing the greying of hair. According to embodiments, the method according to the present invention aims at reverting the color of grey hair. According to particular embodiments, the method according to the present invention aims at improving scalp health.

According to a further aspect, subject matter of the present invention is the use of a composition according to the present disclosure, in particular the use of a scalp-and-hair treatment product according to the present disclosure, such as the use of a hair tonic according to the present disclosure for reducing or preventing the greying of hair and improving scalp health.

The compositions, methods and uses of the present invention are intended for topical administration on the human scalp. The compositions, methods and uses of the present invention are suitable both for cosmetic and therapeutic use.

### DEFINITIONS

As used in the specification and the appended claims, the singular forms "a," "an" and "the" include plural referents unless the context clearly dictates otherwise.

The term "may" in the context of this application means "is permitted to" or "is able to" and is a synonym for the term "can." The term "may" as used herein does not mean possibility or chance.

The term "and/or" in the context of this application means one or the other or both. For example, an aqueous solution of A and/or B means an aqueous solution of A alone, an aqueous solution of B alone and an aqueous solution of a combination of A and B.

The term "about" is understood to mean ±10 percent of the recited number, numbers or range of numbers. According to embodiments, the term "about" is understood to mean ±2 percent of the recited number, numbers or range of numbers.

As used herein, the term "optionally" means that the corresponding step or feature may or may not be present. It includes both possibilities.

The terms "parts-by-weight" (PBW) and "parts-by-volume" (PBV) are used herein in their usual meaning. When used in a mixed context, for example an amount of a component indicated in PBW in admixture with a component indicated in PBV, the terms PBW and PBV as used herein denote a weight amount (for example in grams) of the component indicated in PBW relative to a volume in (for example in millilitres) of the amount of the component indicated in PBV. Such relation "PBW/PBV" may be denoted as "(w/v)".

The terms "ppm" (parts-per-million) and "ppb" (parts-per-billion) are used herein in their usual meaning. Used in the context of weight, "ppm-by-weight" and "ppb-by-weight" are indicative of a concentration. A value of "5 ppm-by-weight" indicates a concentration of 5 milligrams/litre (mg/l), and a value of "5 ppb-by-weight" respectively indicates a concentration of 5 micrograms/litre (µl/l).

Unless specifically indicated otherwise, all values, ranges, percentages etc. in the present disclosure are "by-weight".

The term "composition" as used herein denotes a mixture or blend comprising at least two components. Typical compositions comprise at least one functional component (such as, for example, an active ingredient) and at least one solvent component (such as water or an alcohol). A composition may be present in any suitable physical form, including for example liquids of varying viscosities, gels, foams, creams, and the like. Typically, the compositions according to the present invention are liquids (including solutions and dispersions). The term "composition" may denote a ready-to-use product unless the context dictates otherwise.

The term "extract" as used herein denotes a mixture of at least two different molecules obtained from an indicated plant source. The at least two different molecules in the extract may be (botanical) compounds naturally occurring in the said plant source. The at least two different molecules in the extract may contain, or essentially consist of, or consist of degradation products obtained from compounds naturally occurring in the said plant source. Such degradation products include for example, hydrolyzates, for example hydrolyzates obtained by enzymatic degradation. Such degradation products further include enzymatic degradation products other than hydrolyzates. The term "extract" further denotes that the plant compounds are present (dissolved/dispersed) in a liquid medium not derived from the said plant source, i.e. the plant compounds are dissolved/dispersed a solvent or mixture of solvents.

The liquid medium of the extracts in compositions according to the present invention may differ from the liquid medium originally used to extract compounds from a respective plant source. The liquid medium of the extracts in compositions according to the present invention typically has similar solubility characteristics for plant compounds as the liquid medium previously used to extract the said plant compounds from a respective plant source. In a particular embodiment, the solvent or solvent mixture in extracts present in compositions according to the present invention is/are identical with the solvent or solvent mixture originally used for preparing an extract from a respective plant source.

The terms "solids content" or "dry matter" are used herein in their usual meaning, and they are used interchangeably. As used herein, the two terms denote the material/matter of a plant extract stripped of solvent (including moisture). The "solids content" is determined by LOD (loss-on-drying) using standard procedures. The determination involves removing solvents and moisture, under conditions of decreased pressure and/or increased temperature (below degradation temperatures), until the sample weight remains constant. Conveniently, the "solids content" may be determined using for example a moisture analyser (moisture balance). For the purposes of the present invention, the "solids content" of a respective plant extract is essentially free of, or free of water and solvent(s), for example the solvents(s) originally used to extract compounds from a respective plant source.

"Ready-to-use scalp-and-hair treatment product" denotes the form in which the composition is actually applied to human scalp, or to a skin region comprising facial hair.

The term "essentially free" of a particular component denotes an amount of less than 1.0% by weight of the respective component in an entity such as a composition, based on the total weight of the entity. In particular, the term "essentially free" denotes an amount of less than 0.5% by weight, for example less than 0.1% by weight, such as less than 0.01% by weight of the respective component in the entity.

The term "free" of a particular component denotes an amount below the detection limit, assuming that detectability is on a ppm (parts per million) basis (by weight). In particular, the term "free" of a particular component denotes an amount below 50 ppm-by-weight, for example less than 10 ppm-by-weight, such as less than 5 ppm-by-weight of the respective component in the entity.

The term "consisting essentially of" denotes the absence of additional, not specifically recited components in an entity such as a composition in an amount of more than 1.0% by weight, based on the total weight of the entity. In particular, the term "consisting essentially of" denotes the absence of additional, not specifically recited components in an amount of more than 0.5% by weight, for example more than 0.1% by weight, such as more than 0.01% by weight in an entity. It goes without saying that any additional, not specifically recited component is compatible with the specifically recited components and does not interfere with the function thereof.

The term "consisting of" denotes the absence of additional, not specifically recited components in an entity such as a composition in an amount amount below the detection limit, assuming that detectability is on a ppm (parts per million) basis (by weight). In particular, the term "consisting of" denotes the absence of additional, not specifically recited components in an amount of more than 50 ppm-by-weight, for example more than 10 ppm-by-weight, such as more than 10 ppm-by-weight in an entity. It goes without saying that any additional, not specifically recited component is compatible with the specifically recited components and does not interfere with the function thereof.

The term "hair" as used herein denotes generally mammalian hair, but in particular human hair. More specifically, for the purposes of the present invention the term "hair" denotes human "head hair" or "scalp hair", and human "facial hair". The term "facial hair" encompasses beard hair, eyebrows and eyelashes. In this context, for the purposes of the present invention the term "scalp" is intended to encompass any facial skin region, and "applying to scalp" is intended to encompass applying the compositions of the present invention to (at least part of the) scalp region and/or to (at least part of) any facial skin region.

The term "grey hair" denotes hair having lost at least part of its pigmentation, which loss results in a color that differs from its original color. Grey hair is perceived as greyish or whitish color, and typically due to a decrease of melanin production by melanocytes. Grey hair often is perceived as a negative aesthetic phenomenon, and typically is associated with the natural ageing process.

The term "reducing the greying of hair" denotes a deceleration or retardation of the natural hair greying process. "Preventing the greying of hair" denotes halting the natural hair greying process to an extent that a change of a head's hair color overall is not perceivable after a given time interval. "Reverting the color of grey hair" denotes an improvement of the hair color to a less-grey or less-whitish color, i.e. at least in part back to its original color. "Reverting the color of grey hair" typically is associated with melanin re-pigmentation. Suitable intervals for observing differences in the hair color are at least four weeks, for example an interval of one month, in particular an interval of four months, or longer. Hair color differences are as perceived by the naked eye. If more accuracy is desired, hair color differences may be determined by photography, microscopy, and/or color analysis.

The term "age-related hair-thinning" denotes a perceived loss of the volume of human hair caused by an overall hair loss, and/or by a decrease of the diameter or cross-section of the hair, which decrease occurs in an age-dependent fashion with an onset age typically in the early 40's.

As used herein, the terms "sandal pentanol" and "Sandalore" are used interchangeably. Sandal pentanol is a synthetic odorant having a fragrance similar to sandalwood, and which is used in perfumes, emollients and skin cleaning agents as a less expensive ingredient mimicking sandalwood scent. The IUPAC denomination of sandal pentanol is 3-methyl-5-(2,2,3-trimethylcyclopent-3-en-1-yl)pentan-2-ol.

### DETAILED DESCRIPTION OF THE INVENTION

The inventors of the present invention surprisingly found out that a combination of a plant extract from Avena strigosa (black oat), a plant extract from Ononis spinosa (spiny restharrow), and of 2-O-feruloyl-L-malic acid is suitable for reducing or preventing the greying of hair. It is believed that the active ingredients of the two plant extracts, and the active ingredient 2-O-feruloyl-L-malic acid act in a synergistic manner.

Subject matter of the present invention is a composition comprising an Avena strigosa extract, an Ononis spinosa extract, and 2-O-feruloyl-L-malic acid. The active ingredient 2-O-feruloyl-L-malic acid optionally may be present in the form of a plant extract from Nasturtium officinale (watercress). Typically, the composition also comprises a solvent or solvent-water system.

### 1 Avena strigosa extract

The compositions according to the present invention comprise an Avena strigosa extract as an active ingredient. Avena strigosa (black oat, also called lopsided oat or bristle oat) is a grass species native to Europe. It grows to a height of about 0.8 to 1.5 meters, and has edible seeds. It is a nutritive grass with good protein content, and is cultivated, in particular as a forage crop for ruminants.

The Avena strigosa extract for use in the present invention may be obtained from any part of the plant, including for example leaves, stems, roots, flowers, seeds, fruits, or combinations thereof. Preferably, the extract is obtained from seeds. In particular, the Avena strigosa extract may be obtained by extraction of plant parts, in particular seeds, using a water-based, alcohol-based or water-alcohol-based extraction solvent. According to particular embodiments, the Avena strigosa extract may be obtained by extraction of plant parts with an extraction solvent or solvent system comprising water and an alcohol selected from ethanol, propanol, butanol, pentanol, hexanol, glycerol, ethylene glycol, propylene glycol, butylene glycol, pentylene glycol, or a combination thereof.

The solvent or solvent system of the Avena strigosa extract present in compositions according to the present invention may differ from the liquid medium originally used to extract active ingredients from Avena strigosa plant parts. The solvent or solvent system of the Avena strigosa extract in compositions according to the present invention typically has solubility characteristics for active ingredients, however, which are similar to the ones of the solvent or solvent system originally used to extract active ingredients from Avena strigosa plant parts. In a particular embodiment, the solvent or solvent mixture of the Avena strigosa extract present in compositions according to the present invention is/are identical with the solvent or solvent mixture originally used for extracting active ingredients from Avena strigosa plant parts.

The Avena strigosa extract present in the composition(s) according to the present invention therefore may, analogously, be water-based, alcohol-based or water-alcohol-based. According to particular embodiments, the Avena strigosa extract present in the composition(s) according to the present invention comprises water and an alcohol selected from ethanol, propanol, butanol, pentanol, hexanol, glycerol, ethylene glycol, propylene glycol, butylene glycol, pentylene glycol, or a combination thereof.

The Avena strigosa extract may contain, or essentially consist of, or consist of degradation products obtained from compounds naturally occurring in Avena strigosa. Such degradation products include for example, hydrolyzates, for example hydrolyzates obtained by enzymatic degradation of Avena strigosa. Enzymes useful for preparing hydrolyzates include proteases, for example.

According to embodiments, the Avena strigosa extract is an aqueous glycolic extract, for example an aqueous butylene glycol extract. For instance, the solvent (system) of the Avena strigosa extract may be 50/50 (v/v) water/butylene glycol.

The Avena strigosa extract typically has a solids content in the range of 0.10-16.0 percent-by-weight, in particular a solids content in the range of 0.2-10.0 percent-by-weight. More specifically, the Avena strigosa extract may have a solids content in the range of 0.50-8.0 percent-by-weight, for example a solids content in the range of 0.60-6.0 percent-by-weight.

According to particular embodiments, the Avena strigosa extract comprises peptides having a molecular mass of less than 2.0 kDa. For example, the Avena strigosa extract comprises 15.0-70.0 percent-by-weight, based on the extract's solids content, peptides having a molecular mass of less than 2.0 kDa. In particular, the Avena strigosa extract may comprise 20.0-50.0 percent-by-weight, based on the extract's solids content, peptides having a molecular mass of less than 2.0 kDa. More specifically, the Avena strigosa extract may comprise 25.0-35.0 percent-by-weight, based on the extract's solids content, peptides having a molecular mass of less than 2.0 kDa.

According to particular embodiments, the Avena strigosa extract comprises flavenoids, phenolic acids, and/or polyphenolic compounds. For example, the Avena strigosa extract comprises 15.0-70.0 percent-by-weight, based on the extract's solids content, flavenoids, phenolic acids, and/or polyphenolic compounds. In particular, the Avena strigosa extract may comprise 20.0-50.0 percent-by-weight, based on the extract's solids content, flavenoids, phenolic acids, and/or polyphenolic compounds. More specifically, the Avena strigosa extract may comprise 25.0-35.0 percent-by-weight, based on the extract's solids content, flavenoids, phenolic acids, and/or polyphenolic compounds.

### 2 Ononis spinosa extract

The compositions according to the present invention comprise an Ononis spinosa extract as an active ingredient. Ononis spinosa (spiny restharrow) is a flowering plant native to Europe of the Fabaceae family, with a flowering period from May to October. It is a bushy perennial growing to a height of about 0.6 meters, having woody spiny branches, small and oblong leaves, and butterfly-shaped flowers.

The Ononis spinosa extract for use in the present invention may be obtained from any part of the plant, including for example leaves, stems, roots, flowers, seeds, fruits, or combinations thereof. Preferably, the extract is obtained from the roots. In particular, the Ononis spinosa extract may be obtained by extraction of plant parts, in particular roots, using a water-based, alcohol-based or water-alcohol-based extraction solvent. According to particular embodiments, the Ononis spinosa extract may be obtained by extraction of plant parts with an extraction solvent or solvent system comprising water and an alcohol selected from ethanol, propanol, butanol, pentanol, hexanol, glycerol, ethylene glycol, propylene glycol, butylene glycol, pentylene glycol, or a combination thereof.

The solvent or solvent system of the Ononis spinosa extract present in compositions according to the present invention may differ from the liquid medium originally used to extract active ingredients from Ononis spinosa plant parts. The solvent or solvent system of the Ononis spinosa extract in compositions according to the present invention typically has solubility characteristics for active ingredients, however, which are similar to the ones of the solvent or solvent system originally used to extract active ingredients from Ononis spinosa plant parts. In a particular embodiment, the solvent or solvent mixture of the Ononis spinosa extract present in compositions according to the present invention is/are identical with the solvent or solvent mixture originally used for extracting active ingredients from Ononis spinosa plant parts.

The Ononis spinosa extract present in the composition(s) according to the present invention therefore may, analogously, be water-based, alcohol-based or water-alcohol-based. According to particular embodiments, the Ononis spinosa extract present in the composition(s) according to the present invention comprises water and an alcohol selected from ethanol, propanol, butanol, pentanol, hexanol, glycerol, ethylene glycol, propylene glycol, butylene glycol, pentylene glycol, or a combination thereof.

The Ononis spinosa extract may contain, or essentially consist of, or consist of degradation products obtained from compounds naturally occurring in Ononis spinosa. Such degradation products include for example, hydrolyzates, for example hydrolyzates obtained by enzymatic degradation of Ononis spinosa. Enzymes useful for preparing hydrolyzates include proteases, for example.

According to embodiments, the Ononis spinosa extract is an aqueous glycolic extract, for example an aqueous butylene glycol extract. For instance, the solvent (system) of the Ononis spinosa extract may be 50/50 (v/v) water/butylene glycol.

The Ononis spinosa extract typically has a solids content in the range of 0.10-16.0 percent-by-weight, in particular a solids content in the range of 0.2-10.0 percent-by-weight. More specifically, the Ononis spinosa extract may have a solids content in the range of 0.50-8.0 percent-by-weight, for example a solids content in the range of 0.60-6.0 percent-by-weight

According to particular embodiments, the Ononis spinosa extract comprises peptides having a molecular mass of less than 2.0 kDa. For example, the Ononis spinosa extract comprises 15.0-70.0 percent-by-weight, based on the extract's solids content, peptides having a molecular mass of less than 2.0 kDa. In particular, the Ononis spinosa extract may comprise 20.0-50.0 percent-by-weight, based on the extract's solids content, peptides having a molecular mass of less than 2.0 kDa. More specifically, the Ononis spinosa extract may comprise 25.0-35.0 percent-by-weight, based on the extract's solids content, peptides having a molecular mass of less than 2.0 kDa.

According to particular embodiments, the Ononis spinosa extract comprises flavenoids, phenolic acids, and/or polyphenolic compounds. For example, the Ononis spinosa extract comprises 15.0-70.0 percent-by-weight, based on the extract's solids content, flavenoids, phenolic acids, and/or polyphenolic compounds. In particular, the Ononis spinosa extract may comprise 20.0-50.0 percent-by-weight, based on the extract's solids content, flavenoids, phenolic acids, and/or polyphenolic compounds. More specifically, the Ononis spinosa extract may comprise 25.0-35.0 percent-by-weight, based on the extract's solids content, flavenoids, phenolic acids, and/or polyphenolic compounds.

### 3 2-O-feruloyl-L-malic acid

The compositions according to the present invention comprise 2-O-feruloyl-L-malic acid as an active ingredient. 2-O-feruloyl-L-malic acid may be synthesized from L-malic acid and ferulic acid as starting materials. Alternatively, 2-O-feruloyl-L-malic acid may be obtained, for example, by extracting watercress (Nasturtium officinale). It has the following formula:

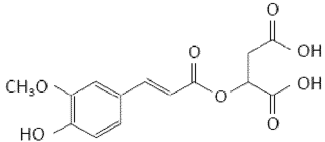

As used herein, the term "2-O-feruloyl-L-malic acid" is intended to denote all optical isomers, racemates, other isomeric forms, and cosmetically acceptable salts thereof (in particular the sodium, potassium, magnesium, calcium and ammonium salts).

2-O-feruloyl-L-malic acid has been identified as a promoter of the stem cell growth factor RSPO1 (R-spondin-1). Further, it is believed that 2-O-feruloyl-L-malic acid has a positive effect in reducing hair loss, and reducing or preventing the greying of hair.

The compositions according to the present invention typically comprise 2-O-feruloyl-L-malic acid in a concentration of 0.0010-20.0 ppm-by-weight, in particular 0.010-2.0 ppm-by-weight. More specifically, the compositions according to the present invention may comprise 2-O-feruloyl-L-malic acid in a concentration of 0.050-0.20 ppm-by-weight, based on the total weight of the composition.

### 4 Nasturtium officinale extract

As noted above, 2-O-feruloyl-L-malic acid may be obtained, for example, by extracting Nasturtium officinale (watercress). Accordingly, the active ingredient 2-O-feruloyl-L-malic acid may be present in compositions according to the present invention in the form of Nasturtium officinale extract.

Nasturtium officinale (watercress, also called yellowcress) is a species of an aquatic flowering plant of the Brassicaceae family. It is a perennial plant native to Asia and Europe.

The Nasturtium officinale extract for use in the present invention may be obtained from any part of the plant, including for example leaves, stems, roots, flowers, seeds, fruits, or combinations thereof. Preferably, the extract is obtained from leaves (or other above-graound parts). In particular, the Nasturtium officinale extract may be obtained by extraction of plant parts, in particular leaves, using a water-based, alcohol-based or water-alcohol-based extraction solvent. According to particular embodiments, the Nasturtium officinale extract may be obtained by extraction of plant parts with an extraction solvent or solvent system comprising water and an alcohol selected from ethanol, propanol, butanol, pentanol, hexanol, glycerol, ethylene glycol, propylene glycol, butylene glycol, pentylene glycol, or a combination thereof.

The solvent or solvent system of the Nasturtium officinale extract present in compositions according to the present invention may differ from the liquid medium originally used to extract active ingredients from Nasturtium officinale plant parts. The solvent or solvent system of the Nasturtium officinale extract in compositions according to the present invention typically has solubility characteristics for active ingredients, however, which are similar to the ones of the solvent or solvent system originally used to extract active ingredients from Nasturtium officinale plant parts. In a particular embodiment, the solvent or solvent mixture of the Nasturtium officinale extract optionally used in compositions according to the present invention is/are identical with the solvent or solvent mixture originally used for extracting active ingredients from Nasturtium officinale plant parts.

The Nasturtium officinale extract optionally used in the composition(s) according to the present invention therefore may, analogously, be water-based, alcohol-based or water-alcohol-based. According to particular examples, the Nasturtium officinale extract optionally used in the composition(s) according to the present invention comprises water and an alcohol selected from ethanol, propanol, butanol, pentanol, hexanol, glycerol, ethylene glycol, propylene glycol, butylene glycol, pentylene glycol, or a combination thereof.

For example, the Nasturtium officinale extract optionally used in the composition(s) according to the present invention therefore may be an aqueous alcohol extract, for example an aqueous ethanol extract. For instance, the solvent (system) of the Nasturtium officinale extract may be 50/50 (v/v) water/ethanol.

The Nasturtium officinale extract may contain, or essentially consist of, or consist of degradation products obtained from compounds naturally occurring in Nasturtium officinale. Such degradation products include for example, hydrolyzates, for example hydrolyzates obtained by enzymatic degradation of Nasturtium officinale. Enzymes useful for preparing hydrolyzates include proteases, for example.

The Nasturtium officinale extract typically has a solids content in the range of 0.10-16.0 percent-by-weight, in particular a solids content in the range of 0.20-10.0 percent-by-weight. More specifically, the Nasturtium officinale extract may have a solids content in the range of 0.50-8.0 percent-by-weight, for example a solids content in the range of 0.60-6.0 percent-by-weight.

The Nasturtium officinale extract may comprise peptides having a molecular mass of less than 2.0 kDa. For example, the Nasturtium officinale extract comprises 15.0-70.0 percent-by-weight, based on the extract's solids content, peptides having a molecular mass of less than 2.0 kDa. In particular, the Nasturtium officinale extract may comprise 20.0-50.0 percent-by-weight, based on the extract's solids content, peptides having a molecular mass of less than 2.0 kDa. More specifically, the Nasturtium officinale extract may comprise 25.0-35.0 percent-by-weight, based on the extract's solids content, peptides having a molecular mass of less than 2.0 kDa.

The Nasturtium officinale extract may comprise flavenoids, phenolic acids, and/or polyphenolic compounds. For example, the Nasturtium officinale extract comprises 15.0-70.0 percent-by-weight, based on the extract's solids content, flavenoids, phenolic acids, and/or polyphenolic compounds. In particular, the Nasturtium officinale extract may comprise 20.0-50.0 percent-by-weight, based on the extract's solids content, flavenoids, phenolic acids, and/or polyphenolic compounds. More specifically, the Nasturtium officinale extract may comprise 25.0-35.0 percent-by-weight, based on the extract's solids content, flavenoids, phenolic acids, and/or polyphenolic compounds.

### 5 Relative amounts of extracts

Compositions according to the present invention suitably may comprise 0.10-10.0 percent-by-weight, based on the total weight of the composition, Avena strigosa extract, and 0.10-10.0 percent-by-weight, based on the total weight of the composition, Ononis spinosa extract. For example, compositions according to the present invention may comprise 0.20-5.0 percent-by-weight, based on the total weight of the composition, Avena strigosa extract, and 0.20-5.0 percent-by-weight, based on the total weight of the composition, Ononis spinosa extract. More specifically, compositions according to the present invention may comprise 0.30 to 3.0 percent-by-weight, based on the total weight of the composition, Avena strigosa extract, and 0.30 to 3.0 percent-by-weight, based on the total weight of the composition, Ononis spinosa extract.

Termed differently, compositions according to the present invention suitably may comprise 20.0 to 4000.0 ppm-by-weight, based on the total weight of the composition, Avena strigosa extract, and 20.0 to 4000.0 ppm-by-weight, based on the total weight of the composition, Ononis spinosa extract. For example, compositions according to the present invention may comprise 50.0 to 2000.0 ppm-by-weight, based on the total weight of the composition, Avena strigosa extract, and 50.0 to 2000.0 ppm-by-weight, based on the total weight of the composition, Ononis spinosa extract. More specifically, compositions according to the present invention may comprise 100.0 to 800.0 ppm-by-weight, based on the total weight of the composition, Avena strigosa extract, and 100.0 to 800.0 ppm-by-weight, based on the total weight of the composition, Ononis spinosa extract.

If the active ingredient 2-O-feruloyl-L-malic acid is present in the form of Nasturtium officinale extract, compositions according to the present invention suitably may comprise 0.20-20.0 percent-by-weight, based on the total weight of the composition, Nasturtium officinale extract. For example, compositions according to the present invention may comprise 0.40-10.0 percent-by-weight, based on the total weight of the composition, Nasturtium officinale extract. More specifically, compositions according to the present invention may comprise 0.80-5.0 percent-by-weight, based on the total weight of the composition, Nasturtium officinale extract.

Again termed differently, if the active ingredient 2-O-feruloyl-L-malic acid is present in the form of Nasturtium officinale extract, compositions according to the present invention suitably may comprise 2.0 to 800.0 ppm-by-weight, based on the extract's solids content, Nasturtium officinale extract. For example, compositions according to the present invention may comprise 4.0 to 400.0 ppm-by-weight, based on the total weight of the composition, Nasturtium officinale extract. More specifically, compositions according to the present invention may comprise 16.0 to 100.0 ppm-by-weight, based on the extract's solids content, Nasturtium officinale extract.

Particular examples of compositions according to the present invention comprise the extracts of Avena strigosa and Ononis spinosa at a weight ratio of Avena strigosa extract to Ononis spinosa extract in the range of 0.20-5.0. For example, the weight ratio of Avena strigosa extract to Ononis spinosa extract may be in the range of 0.50-2.0. More specifically, the weight ratio of Avena strigosa extract to Ononis spinosa extract may be in the range of 0.80-1.20.

Compositions according to the present invention comprising an extract of Nasturtium officinale may exhibit a weight ratio of Avena strigosa extract to Ononis spinosa extract to Nasturtium officinale extract is in the range of [0.20-5.0] : [0.20-5.0] : [0.40-8.0]. For example, a weight ratio of Avena strigosa extract to Ononis spinosa extract to Nasturtium officinale extract is in the range of [0.60-2.0] : [0.60-2.0] : [0.80-4.0]. According to a specific example of a compositions according to the present invention, the weight ratio of Avena strigosa extract to Ononis spinosa extract to Nasturtium officinale extract may be in the range of [0.80-1.20] : [0.80-1.20] : [1.0-2.5].

### 6 Commercially available extracts

A mixed extract of Avena strigosa and Ononis spinosa, suitable for use in compositions according to the present invention, is available from Silab (Z.I. de la Nau, 19240 Saint-Viance, France) under the product name Agreynist^{®}. It is believed that that Agreynist^{®} comprises about 0.50-2.0 percent-by-weight, based on the extract's solids content, Avena strigosa extract, and about 0.50-2.0 percent-by-weight, based on the extract's solids content, Ononis spinosa extract, in an aqueous butylene glycol solvent system.

If the Avena strigosa extract and the Ononis spinosa extract of the compositions according to the present invention are provided in the form of the commercial product Agreynist^{®}, the total amount of Agreynist^{®} (constituting both Avena strigosa extract and Ononis spinosa extract) suitably may be in the range of 0.50-12.0 percent-by-weight, based on the total weight of the composition, and typically may be in the range of 0.80-6.0 percent-by-weight, based on the total weight of the composition. According to specific embodiments, the compositions according to the present invention may comprise Agreynist^{®} (constituting both Avena strigosa extract and Ononis spinosa extract) in a total amount in the range of 1.20-4.0 percent-by-weight, based on the total weight of the composition.

An extract of Nasturtium officinale, suitable for use in compositions according to the present invention, is available from Ichimaru Pharcos Co., Ltd. (Gifu, Japan) under the product name Burgeon-Up. It is believed that that Burgeon-Up comprises about 0.050-0.50 percent-by-weight, based on the extract's solids content, Nasturtium officinale extract, in an aqueous ethanolic solvent system.

If the active ingredient 2-O-feruloyl-L-malic acid is provided in compositions according to the present invention in the form of Nasturtium officinale extract, and the Nasturtium officinale extract is provided in the form of the commercial product Burgeon-Up, the total amount of Burgeon-Up in compositions according to the present invention suitably may be in the range of 0.40-10.0 percent-by-weight, based on the total weight of the composition, and typically may be in the range of 0.80-5.0 percent-by-weight, based on the total weight of the composition. According to specific embodiments, the compositions according to the present invention may comprise Agreynist^{®} (constituting both Avena strigosa extract and Ononis spinosa extract) in a total amount in the range of 1.20-3.50 percent-by-weight, based on the total weight of the composition.

### 7 Optional further active ingredients

Compositions according to the present disclosure optionally may further comprise as additional active ingredients:
- sandal pentanol,
- caffeine,
- one or more saturated and/or unsaturated C10-C18 fatty acids.

### 7.1 Sandal pentanol

Sandal pentanol is an active ingredient optionally used according to the present disclosure. Sandal pentanol is a synthetic odorant used as an ingredient mimicking sandalwood scent. Sandal pentanol is an alcohol having a chemical structure quite distinct from the ingredients of natural sandalwood oil which usually are extracted by steam distillation of wood from matured sandalwood trees. The IUPAC denomination of sandal pentanol is 3-methyl-5-(2,2,3-trimethylcyclopent-3-en-1-yl)pentan-2-ol. Sandal pentanol has been shown to have a stimulatory effect on the olfactory receptor OR2AT4, which is expressed in the epithelium of human hair follicles.

If present, the sandal pentanol typically will be present in an amount of from 0.010 to 10.0 percent-by-weight, based on the weight of the composition. More specifically, if used, the sandal pentanol may be present in an amount of 0.050 to 5.0 percent-by-weight, based on the weight of the composition, or in an amount of 0.10 to 2.0 percent-by-weight, based on the weight of the composition. According to even more specific examples, if used in the compositions according to the present invention, the sandal pentanol may be present in an amount of 0.50 to 1.50 percent-by-weight, based on the weight of the composition.

### 7.2 Caffeine

Caffeine is an optional active ingredient used according to the present disclosure. Caffeine is a plant alkaloid also known under the names theine, methyltheobromine and guaranine. The IUPAC denomination is 1,3,7-trimethylxanthine. Caffeine is a well-known stimulant which is present for example in coffee, black tea, but also in cocoa solids and products containing same (in particular dark chocolate), or cola nuts. Caffeine has a vasodilating effect, thereby increasing blood and nutrients supply to the hair roots. Studies have shown that caffeine enhances the hair shaft elongation, prolonges anagen duration (increasing the hair prowth phase) and stimulates the proliferation of hair matrix keratinocytes (increasing keratin production).

If present, the caffeine typically will be present in an amount of from 0.010 to 10.0 percent-by-weight, based on the weight of the composition. More specifically, if used, the caffeine may be present in an amount of 0.050 to 5.0 percent-by-weight, based on the weight of the composition, or in an amount of 0.10 to 2.0 percent-by-weight, based on the weight of the composition. According to even more specific examples, if used in the compositions according to the present invention, the caffeine may be present in an amount of 0.50 to 1.5 percent-by-weight, based on the weight of the composition.

### 7.3 Fatty acid(s)

Another optional active ingredient used according to the present disclosure are C10-C18 fatty acids. The fatty acid(s) optionally used is/are one or more linear and/or branched, saturated and/or unsaturated C10-C18 fatty acids. Typically, the one or more C10-C18 fatty acids will be linear fatty acids. For example, the C10-C18 fatty acid(s) may be selected from capric acid (C10:0), lauric acid (C12:0), myristic acid (C14:0), isomyristic acid (C14:0), palmitic acid (C16:0), palmitoleic acid (C16:1), stearic acid (C18:0), oleic acid (C18:1), linoleic acid (C18:2), linolenic acid (C18:3), parinaric acid (C18:4), petroselinic acid (C18:1), or combinations thereof.

According to examples, the C10-C18 fatty acid(s) may comprise lauric acid (C12:0), myristic acid (C14:0), or a combination thereof. According to specific examples, the C10-C18 fatty acid(s) may be selected from lauric acid (C12:0), myristic acid (C14:0), or a combination thereof. More specifically, the fatty acid component may be lauric acid (C12:0).

According to examples, the fatty acid component may comprise one or more unsaturated fatty acids. According to specific examples, the C10-C18 fatty acid(s) may comprise palmitoleic acid (C16:1), oleic acid (C18:1), linoleic acid (C18:2), linolenic acid (C18:3), parinaric acid (C18:4), petroselinic acid (C18:1), or a combination thereof. More specifically, the fatty acid component may comprise linoleic acid (C18:2).

According to specific examples, the fatty acid component may be selected from palmitoleic acid (C16:1), oleic acid (C18:1), linoleic acid (C18:2), linolenic acid (C18:3), parinaric acid (C18:4), petroselinic acid (C18:1), or a combination thereof. For example, the fatty acid component may be selected from palmitoleic acid (C16:1) and/or linoleic acid (C18:2).

If the fatty acid component comprises one or more unsaturated fatty acids, the respective compositions preferably comprise an antioxidant in order to reduce autoxidation of the double bonds.

According to other examples, compositions according to the present disclosure may be essentially free, or free of (added) unsaturated C10-C18 fatty acids. According to specific examples, compositions according to the present disclosure may be essentially free, or free of unsaturated fatty acids per se.

If present, the C10-C18 fatty acid(s) may be present in the form of free acid, or in the form of a cosmetically acceptable salt, for example in the form of an alkali metal salt, alkaline earth metal salt, ammonium salt or combination thereof. For example, if present the C10-C18 fatty acid(s) may be present in the form of the free acid(s).

If present, the C10-C18 fatty acid(s) typically will be present in an amount of from 0.010 to 10.0 percent-by-weight, based on the weight of the composition. More specifically, if used, the C10-C18 fatty acid(s) may be present in an amount of 0.050 to 5.0 percent-by-weight, based on the weight of the composition, or in an amount of 0.10 to 2.0 percent-by-weight, based on the weight of the composition. According to even more specific examples, if used in the compositions according to the present invention, the C10-C18 fatty acids may be present in an amount of 0.50 to 1.5 percent-by-weight, based on the weight of the composition.

### 7.4 Relative amounts of sandal pentanol, caffeine, and fatty acid(s) in combination

If present, sandal pentanol typically will be present in an amount of from 0.010 to 10.0 percent-by-weight, based on the weight of the composition. Caffeine, if present, typically will be present in an amount of from 0.010 to 10.0 percent-by-weight, based on the weight of the composition. One or more C10-C18 fatty acid(s), if present, typically will be present in an amount of from 0.010 to 2.0 percent-by-weight, based on the weight of the composition. Conveniently, the sandal pentanol will be present in an amount of from 0.10 to 5.0 percent-by-weight, the caffeine will be present in an amount of from 0.10 to 5.0 percent-by-weight, and the one or more C10-C18 fatty acid(s) will be present in an amount of from 0.050 to 3.0 percent-by-weight, each based on the weight of the composition.

For example, the sandal pentanol may be present in an amount of from 0.50 to 2.0 percent-by-weight, the caffeine may be present in an amount of from 0.50 to 2.0 percent-by-weight, and the one or more C10-C18 fatty acid(s) may be present in an amount of from 0.10 to 2.0 percent-by-weight, each based on the weight of the composition. According to more specific examples, the sandal pentanol, if used, will be present in an amount of from 0.80 to 1.50 percent-by-weight, the caffeine, if used, will be present in an amount of from 0.80 to 1.50 percent-by-weight, and the one or more C10-C18 fatty acid(s) , if used, will be present in an amount of from 0.10 to 1.0 percent-by-weight, each based on the weight of the composition.

For compositions according to the present invention comprising the combination of sandal pentanol, caffeine and/or one or more C10-C18 fatty acid(s), the weight ratio of sandal pentanol to caffeine may be from 1:2 to 2:1, more specifically from 1:1.5 to 1.5:1, and in particular from 1:1.2 to 1.2:1. In such embodiments, the weight ratio of sandal pentanol to the one or more C10-C18 fatty acid(s) may be from 10:1 to 1:1, more specifically from 8:1 to 1.5:1, and in particular from 5:1 to 2:1. Further, the weight ratio of caffeine to the one or more C10-C18 fatty acid(s) analogously may be from 10:1 to 1:1, more specifically from 8:1 to 1.5:1, and in particular from 5:1 to 2:1. Given that the molecular weights of sandal pentanol, caffeine and C10-C18 fatty acids are rather similar, molecular ratios will be similar to the weight ratios.

### 8 Optional added-benefit components

Compositions according to the present disclosure may optionally comprise additional components which enhance, supplement or add to the benefit obtained by sandal pentanol alone or an active ingredient combination. Such additional optional components will be referred to in the following as supportive component, oil component and mineral component. The supportive component covers a broad spectrum of substances having diversified influences on physiologic or metabolic processes. The oil component covers natural essential oils and modified oils. Modified oils include in particular hydrogenated oils and oil constituents. The mineral component covers minerals in water-soluble or alcohol-soluble form.

According to embodiments, compositions according to the present disclosure further may comprise a supportive component. Examples of such supportive components include in particular vasodilatators, blood circulation stimulators, antioxidants, vitamins, provitamins. If present, the amount of the supportive component or combinations thereof will be in the range of from 0.0010 to 5.0, in particular from 0.010 to 3.0 percent-by-weight, based on the weight of the composition.

The supportive component may comprise, for example, one or more of tocopherols, tocotrienols, tocopherol esters, nicotinic acid, nicotinic amides, nicotinic esters, vasodilatory plant extracts, camphor, royal jelly, biotin, vitamin B3, vitamin B12, vitamin C, vitamin D2, vitamin D3, vitamin E, vitamin K1, vitamin K2, panthenol (provitamin B5), omega-3 fatty acids, omega-6 fatty acids, folates, cannabidiol, amino acids, or combinations thereof.

According to embodiments, the supportive component includes a vasodilator selected from tocopherols (alpha-, beta-, gamma- or delta-tocopherol), in particular alpha-tocopherol, tocopherol esters (including, for example, tocopherol acetate, tocopheryl succinate, tocopherylnicotinate, tocopherylpoly(oxyethylene)-succinate), nicotinic acid, nicotinic amides, nicotinic esters, camphor; an antioxidant such as vitamin C or polyphenol(s); an inhibitor of testosterone-5-alpha-reductase such as the omega-6 fatty acid arachidonic acid; a substance providing a benefit to hair health such as nicotinic amide (niacinamide); or a combination thereof. According to embodiments, the supportive component includes tocopherol or tocopheryl acetate (vitamin E acetate), and/or niacinamide. Suitable vasodilatory plant extracts include burned or distilled nettle extract (Urtica Diocia), roast chestnut extract (Aesculus Hippocastanum), Arnica extract (Arnica Montana), or hemp extract.

The oil component may comprise, for example, peppermint oil, rosemary essential oil, lavender essential oil, tea tree oil, ylang-ylang, thyme oil, cedarwood oil, clary sage essential oil, bergamot oil, jojoba oil, sunflower oil, hydrogenated ethylhexyl olivate, or a combination thereof. If present, the oil component will be present in the compositions of the present disclosure in an amount of from 0.0010 to 1.0, in particular from 0.010 to 0.50 percent-by-weight, based on the weight of the composition.

According to embodiments, the oil component includes peppermint oil and/or hydrogenated ethylhexyl olivate.

The mineral component includes metal ions having a beneficial effect on hair and/or scalp health. The mineral component may comprise, for example, iron, zinc, or a combination thereof. If present, the mineral component will be present in the compositions of the present disclosure in 0.0050-0.50 molar concentration, for example in 0.010-0.25 molar concentration.

Compositions according to the present disclosure may comprise a supportive component, an oil component or a mineral component alone or in any combination.

### 9 The solvent or carrier system, and pH range

The solvent system used in the compositions of the present disclosure is water-based, alcohol-based or water-alcohol-based. Typically, the solvent comprises water and one or more monovalent and/or polyvalent C1-C4 alcohols. According to embodiments, the solvent comprises water and an alcohol selected from ethanol, propanol, butanol, pentanol, hexanol, glycerol, ethylene glycol, propylene glycol, butylene glycol, pentylene glycol or a combination thereof.

The solvent further may comprise one or more solubilisation enhancers. The solubilisation enhancers obviously are intended to solubilize compounds which are not or only partially soluble in the solvent at the concentration used in the compositions. Solubilisation enhancers generally include surfactants. Surfactants include anionic, cationic, amphoteric and non-ionic surfactants.

The anionic surfactants may be selected from the group consisting of salts (such as alkaline salts, for example, sodium salts, ammonium salts, amine salts, amino alcohol salts and magnesium salts) of the following compounds: alkyl sulphates, alkyl ether sulphates, alkylamido ether sulphates, alkylarylpolyether sulphates, monoglyceride sulphates; alkyl sulphonates, alkyl phosphates, alkylamide sulphonates, alkylaryl sulphonates, alpha-olefin sulphonates, paraffin sulphonates; alkyl sulphosuccinates, alkyl ether sulphosuccinates, alkylamide sulphosuccinates; alkyl sulphosuccinamates; alkyl sulphoacetates; alkyl ether phosphates; acyl sarcosinates; acyl isethionates; N-acyltaurates; and mixtures thereof. The alkyl or acyl radical of all of these various compounds, for example, comprises from 8 to 24 carbon atoms, and the aryl radical, for example, is chosen from phenyl and benzyl groups. Weakly anionic surfactants can also be used, such as alkyl-D-galactosiduronic acids and their salts, as well as polyoxyalkylenated (C₆-C₂₄) alkyl ether carboxylic acids, polyoxyalkylenated (C₆-C₂₄) alkylaryl ether carboxylic acids, polyoxyalkylenated (C₆-C₂₄) alkylamido ether carboxylic acids and their salts, for example, those comprising from 2 to 50 ethylene oxide groups, and mixtures thereof. Anionic derivatives of polysaccharides, for example carboxyalkyl ether of alkyl polyglucosides, can be also used.

Suitable anionic surfactant(s) may comprise at least one anionic functional group at their head selected from sulfate, sulfonate, phosphate and carboxylates. Fatty acid anionic surfactant(s), if present, would be considered as an optional active ingredient, in order to avoid potential overlap.

Suitable alkyl sulfates include ammonium lauryl sulfate, sodium lauryl sulfate (sodium dodecyl sulfate, SLS, or SDS), and alkyl-ether sulfates, such as sodium laureth sulfate (sodium lauryl ether sulfate or SLES), and sodium myreth sulfate.

Further suitable anionic surfactants may include docusate (dioctyl sodium sulfosuccinate), alkylaryl ether phosphate, alkyl ether phosphate, sodium lauroyl sarcosinate, ammonium laureth sulfate, disodium lauryl sulfosuccinate, and sodium lauryl sulphoacetate.

Preferred anionic surfactants may be selected from the group consisting of sodium laurylethersulfate, sodium laurethethersulfate, sodium dodecyl sulfate, ammonium laurethethersulfat, ammonium dodecyl sulfate, alkylbenzenesulfonate, and combinations thereof.

The surfactant may be a non-ionic surfactant. The non-ionic surfactant may be selected from the group consisting of lanolin alcohol, and polyoxyethylene ethers of fatty alcohols, and mixtures thereof. The non-ionic surfactant may be preferably ceteareth-n, wherein n is from 2 to 100, or from 10 to 30. Suitable nonionic surfactants are compounds that are well known (see, for example, in this respect "Handbook of Surfactants" by M. R. Porter, published by Blackie & Son (Glasgow and London), 1991, pp. 116-178).

Preferred surfactants encompass non-ionic surfactants, in particular polyoxyethylated fatty alcohols or oils. Particularly preferred non-ionic surfactants include polyoxyethylated Castor Oil and hydrogenated polyoxylated Castor Oil. Specific examples of non-ionic surfactants that may be used in the compositions of the present disclosure are PEG-35 Castor Oil, PEG-40 Hydrogenated Castor Oil, and combinations thereof.

Compositions according to the present disclosure typically have a pH within the range of from 3.0 to 8.0. More particularly, compositiona according to the present invention may have a pH within the range of from 4.0 to 6.50, for example within the range of from 4.50 to 5.50.

### 10 Optional cosmetically acceptable additives

Compositions according to the present disclosure optionally may comprise cosmetically acceptable additives, which may contribute to achieve or enhance the properties or effects sought. According to embodiments, the cosmetically acceptable additives may be selected from perfumes, opacifiers, preservatives, pH modifiers, chelators, hair-care materials or skin-care materials, physiologically compatible silicone derivative compounds, light protecting agents, anti-flaking agents, hair luster-imparting agents, combability improving agents, defatting agents, moisturizers, conditioning agents, viscosity modifiers, cooling agents. The cosmetically acceptable additives may be used in any combinations, as desired or required.

Suitable cosmetically acceptable additives not specifically described below are listed in the International Cosmetics Ingredient Dictionary and Handbook, (8th ed.; The Cosmetics, Toiletry, and Fragrance Association). Particularly, vol. 2, sections 3 (Chemical Classes) and 4 (Functions), which are useful in identifying specific additives to achieve a particular purpose or multipurpose. A few of these additives are discussed below, whose disclosure is of course non-exhaustive.

Suitable amount ranges for perfumes, opacifiers, preservatives, pH modifiers, chelators, hair-care materials or skin-care materials, physiologically compatible silicone derivative compounds, light protecting agents, anti-flaking agents, hair luster-imparting agents, combability improving agents, defatting agents, moisturizers, conditioning agents, viscosity modifiers, cooling agents are indicated for additives discussed below. These cosmetically acceptable additives may be present in a total amount of up to 20.0, in particular up to 15.0 percent-by-weight, based on the weight of the composition. Typically, the total amount of these cosmetically acceptable additives may be within a range of from 0.010 to 10.0, conveniently within a range of from 0.010 to 7.0 percent-by-weight, based on the weight of the composition. For example, the total amount of these cosmetically acceptable additives may be within a range of from 0.010 to 5.0, conveniently within a range of from 0.010 to 3.0 percent-by-weight, based on the weight of the composition.

### 10.1 Perfume or fragrance

Compositions according to the present disclosure may comprise a perfume or fragrance. For example, the compositions may comprise from 0.0010 to 1.50, in particular 0.010-0.50 percent-by-weight perfume, based on the weight of the composition. Perfume can provide an enhanced user experience by making the composition smell pleasant and/or invoke emotions, such as relaxing or exciting smells.

Alternatively, compositions according to the present disclosure may be substantially free of perfume and/or fragrance. Some consumers prefer perfume-free compositions.

The perfume may be an animal fragrance or a plant fragrance. The animal fragrance may be selected from the group consisting of musk oil, civet, castoreum, ambergris, and mixtures thereof.

The plant fragrance may be selected from the group consisting of nutmeg extract, cardomon extract, ginger extract, cinnamon extract, patchouli oil, geranium oil, orange oil, mandarin oil, orange flower extract, cedarwood, vetyver, lavandin, ylang extract, tuberose extract, sandalwood oil, bergamot oil, rosemary oil, spearmint oil, peppermint oil, lemon oil, lavender oil, citronella oil, chamomille oil, clove oil, sage oil, neroli oil, labdanum oil, eucalyptus oil, verbena oil, mimosa extract, narcissus extract, carrot seed extract, jasmine extract, olibanum extract, rose extract, and mixtures thereof.

The perfume may comprise one or more scents selected from the group consisting of acetophenone, adoxal, aldehyde C-12, aldehyde C-14, aldehyde C-18, allyl caprylate, ambroxan, amyl acetate, dimethylindane derivatives, α-amylcinnamic aldehyde, anethole, anisaldehyde, benzaldehyde, benzyl acetate, benzyl alcohol and ester derivatives, benzyl propionate, benzyl salicylate, borneol, butyl acetate, camphor, carbitol, cinnamaldehyde, cinnamyl acetate, cinnamyl alcohol, cis-3-hexanol and ester derivatives, cis-3-hexenyl methyl carbonate, citral, citronnellol and ester derivatives, cumin aldehyde, cyclamen aldehyde, cyclo galbanate, damascones, decalactone, decanol, estragole, dihydromyrcenol, dimethyl benzyl carbinol, 6,8-dimethyl-2-nonanol, dimethyl benzyl carbinyl butyrate, ethyl acetate, ethyl isobutyrate, ethyl butyrate, ethyl propionate, ethyl caprylate, ethyl cinnamate, ethyl hexanoate, ethyl valerate, ethyl vanillin, eugenol, exaltolide, fenchone, fruity esters such as ethyl 2-methyl butyrate, galaxolide, geraniol and ester derivatives, helional, 2-heptonone, hexenol, α-hexylcinnamic aldehyde, hydroxycitrolnellal, indole, isoamyl acetate, isoeugenol acetate, ionones, isoeugenol, isoamyl iso-valerate, iso E super, limonene, linalool, lilial, linalyl acetate, lyral, majantol, mayol, melonal, menthol, p-methylacetophenone, methyl anthranilate, methyl cedrylone, methyl dihydrojasmonate, methyl eugenol, methyl ionone, methyl-α-naphthyl ketone, methylphenylcarbinyl acetate, mugetanol, γ-nonalactone, octanal, phenyl ethyl acetate, phenyl-acetaldehyde dimethyl acetate, phenoxyethyl isobutyrate, phenyl ethyl alcohol, pinenes, sandalore, santalol, stemone, thymol, terpenes, triplal, triethyl citrate, 3,3,5-trimethylcyclohexanol, γ-undecalactone, undecenal, vanillin, veloutone, verdox, and mixtures thereof.

### 10.2 Opacifiers or turbidity-inducing agents

Compositions in accordance with the present disclosure may comprise an opacifier, which sometimes is denoted as well by the term "turbidity-inducing agent". For example, the compositions may comprise from 0.10 to 5.0, in particular 0.50-2.0 percent-by-weight opacifier, based on the weight of the composition. Suitable opacifiers include mineral pigments such as, for example, titanium dioxide, alumina, calcium carbonate. Other suitable opacifiers include fatty alcohols and fatty acid esters such as, for example, cetearyl alcohol, cetyl alcohol, ethylene glycol distearate, glyceryl stearate, glyceryl oleate, stearyl stearate.

### 10.3 Preservatives

Compositions in accordance with the present disclosure may comprise a preservative or mixture of preservatives. The compositions may comprise the preservative(s) in an amount of up to 1.0, for example from 0.0010 to 1.0, in particular from 0.010 to 0.50 percent-by-weight, based on the weight of the composition. Cosmetically acceptable preservatives include organic acids such as para-hydroxybenzoic acid; organic acid salts such as sodium benzoate; compounds such as benzyl alcohol, phenoxyethanol, 1,3-bis(hydroxymethyl)-5,5-dimethylimidazolidine-2,4-dione; and mixtures thereof.

### 10.4 pH modifiers

Compositions in accordance with the present disclosure may comprise a pH modifier and/or buffering agent, in a sufficient amount to effectively adjust the pH of the composition. For example, the compositions may comprise up to 1.0, for example 0.0010-0.50 percent-by-weight pH modifiers, based on the weight of the composition. Suitable pH modifiers and/or buffering agents for use herein include, but are not limited to: ammonia, alkanolamines such as monoethanolamine, diethanolamine, triethanolamine, monopropanolamine, dipropanolamine, tripropanolamine, tripropanolamine, 2-amino-2-methyl-1-propanol, and 2-amino-2-hydroxymethyl-1,3,-propandiol and guanidium salts, alkali metal and ammonium hydroxides and carbonates, such as sodium hydroxide, sodium silicate, sodium meta silicate and ammonium carbonate, and acids such as organic and inorganic acids, for example ascorbic acid, citric acid, acetic acid or tartaric acid, phosphoric acid, hydrochloric acid, and mixtures thereof.

Preferred alkaline pH modifiers include ammonia, ethanolamines, and alkali metal hydroxides, in particular sodium hydroxide. Preferred acidic pH modifiers include hydrochloric acid, ascorbic acid, or citric acid.

### 10.5 Chelators

Compositions according to the present disclosure may further comprise one or more chelators (also known as "chelating agent", "sequestering agent", or "sequestrant") in an amount sufficient to reduce the amount of metals available to interact with formulation components. Chelators are well known in the art and a non-exhaustive list thereof can be found in AE Martell & RM Smith, Critical Stability Constants, Vol. 1, Plenum Press, New York & London (1974) and AE Martell & RD Hancock, Metal Complexes in Aqueous Solution, Plenum Press, New York & London (1996). Typically, the compositions may comprise one or more chelators in a total amount of from 0.0010 to 1.0, in particular from 0.010 to 0.50 percent-by-weight, based on the weight of the composition.

The one or more chelators may be selected from the group consisting of carboxylic acids (such as aminocarboxylic acids), phosphonic acids (such as aminophosphonic acids), polyphosphoric acids (such as linear polyphosphoric acids), their salts thereof, and mixtures thereof.

By "salts thereof", it is meant - in the context of chelators - all salts comprising the same functional structure as the chelators they are referring to and including alkali metal salts, alkaline earth salts, ammonium salts, substituted ammonium salts, and mixtures thereof; alternatively sodium salts, potassium salts, calcium salts, magnesium salts, ammonium salts, and mixtures thereof; alternatively monoethanolammonium salts, diethanolammonium salts, triethanolammonium salts, and mixtures thereof.

The one or more chelators may be one or more aminocarboxylic acid chelators comprising one or more carboxylic acid moieties (-COOH) and one or more nitrogen atoms. The one or more aminocarboxylic acid chelators may be selected from the group consisting of diethylenetriamine pentaacetic acid (DTPA), ethylenediamine disuccinic acid (EDDS), ethylenediamine-N,N'-diglutaric acid (EDDG), 2-hydroxypropylenediamine-N-N'-disuccinic acid (HPDDS), glycinamide-N,N'-disuccinic acid (GADS), ethylenediamine-N-N'-diglutaric acid (EDDG), 2-hydroxypropylenediamine-N-N'-disuccinic acid (HPDDS), ethylenediaminetetraacetic acid (EDTA), ethylenedicysteic acid (EDC), ethylenediamine-N-N'-bis(ortho-hydroxyphenyl acetic acid) (EDDHA), diaminoalkyldi(sulfosuccinic acids) (DDS), N,N'-bis(2-hydroxybenzyl)ethylenediamine-N,N'-diacetic acid (HBED), their salts thereof, and mixtures thereof. Conveniently, a chelator used in compositions according to the present disclosure, if any, may be EDTA.

### 10.6 Viscosity modifiers or rheology modifiers

Compositions in accordance with the present disclosure may comprise viscosity modifiers, referred to in the art as well as "thickening agents". If present, such viscosity modifiers typicall wil be used in the compositions in an amount of from 0.010 to 3.0, in particular from 0.50 to 1.0 percent-by-weight, based on the weight of the composition. Suitable cosmetically acceptable viscosity modifiers include non-ionic polymers, for example polysaccharides such as cellulose, cellulose gum or xanthan gum, and non-ionic viscosity modifiers based on polyether-1 such as the commercially available viscosity modifiers Pure Thix 1442.

The viscosity of compositions according to the present disclosure may vary over a broad range, in particular depending on the type of consumer product. Generally, compositions according to the present disclosure suitably have a viscosity within the range of less than 0.50 mPa•s to more than 10.0 Pa•s. Hair tonics, for example typically exhibit a viscosity near the lower end of the above range, for example a viscosity within the range of 0.50 mPa•s to 0.10 Pa•s. Other consumer products may exhibit higher viscosities.

### 10.7 Further cosmetically acceptable additives

Compositions in accordance with the present disclosure may further comprise care materials, such as hair-care providing or skin-care-providing plant or vegetable extracts, cationic polymers or cationic surfactants. Typical amounts of care materials in the compositions according to the present disclosure will be in the range of from 0.010 to 5.0, in particular 0.050-2.0 percent-by-weight, based on the weight of the composition.

Additional cosmetically acceptable aditives, which may be present in the compositions according to the present disclosure include:
- physiologically compatible silicone derivative compounds, such as volatile or non-volatile silicone or high molecular weight siloxane polymers, such as for example Quaternium-80, typically in an amount of from 0.05 to 20 percent-by-weight, in particular from 0.10 to 5.0 percent-by-weight, based on the weight of the composition
- light protecting agents, such as for example octylmethoxy cinnamate
- anti-flaking agents, typically in an amount of about 0.01 to 2 percent-by-weight
- hair luster-imparting agents, such as for example phenyltrimethicone,
- combability improving agents, such as for example cationic surfactants such as Behentrimonium Chloride, or cationic polymers such as Polyquaternium-10
- defatting agents
- conditioning agents
- moisturizing agents, such as for example sodium lactate, sodium PCA, glycine, fructose, urea, niacinamide, inositol
- cooling agents, such as for example menthol, eucalptol, geraniol, linalool, isopulegol, cubedol, p-mentane-3,8-diols, hydroxy-citronellal as well as esters and derivatives thereof; ketones such as menthone and carvone; carboxamides, or evaporative cooling agents

The cosmetically acceptable additives may be used in the compositions according to the present disclosure in any combinations, as desired or required.

### 11 Substances potentially excluded

Compositions according to the present disclosure may be essentially free of, or be free of certain substances or classes of chemical compounds. The discussion below is intended to exclude deliberately adding the indicated compounds to a composition according to the present disclosure. As far as compounds indicated below should be present in Avena strigosa extract, Ononis spinosa extract, or Nasturtium officinale extract, the following exclusions shall not apply.

According to embodiments, compositions according to the present disclosure may be essentially free of added biotin. According to embodiments, compositions according to the present disclosure may be free of added biotin. According to particular embodiments, the concentration of added biotin may be less than 5 ppm-by-weight.

According to embodiments, compositions according to the present disclosure may be essentially free of added quaternary ammonium compounds. According to embodiments, compositions according to the present disclosure may be free of added quaternary ammonium compounds. According to particular embodiments, the concentration of added quaternary ammonium compounds may be less than 5 ppm-by-weight.

According to embodiments, compositions according to the present disclosure may be essentially free of added silicones. According to embodiments, compositions according to the present disclosure may be free of added silicones. According to particular embodiments, the concentration of added silicones may be less than 5 ppm-by-weight.

According to embodiments, compositions according to the present disclosure may be essentially free of added quaternary ammonium compounds and added silicones. According to embodiments, compositions according to the present disclosure may be free of added quaternary ammonium compounds and added silicones. According to particular embodiments, the concentration of added quaternary ammonium compounds and added silicones may be less than 5 ppm-by-weight.

According to embodiments, any added polymeric compounds, if present in compositions according to the present disclosure, exclusively are film-forming polymers. In other words, according to such embodiments, compositions according to the present disclosure may be essentially free of added non-film-forming polymers. According to embodiments, compositions according to the present disclosure may be free of added non-film-forming polymers. According to particular embodiments, the concentration of added non-film-forming polymers may be less than 5 ppm-by-weight.

According to embodiments, compositions according to the present disclosure may be essentially free of or be free of added non-film-forming polymers selected from acrylates/Steareth-20 methacrylate copolymer, carbomer, polyacrylamide, hydroxyethylcellulose, and polymers having similar chemistry.

According to embodiments, compositions according to the present disclosure may be essentially free of or be free of added unsaturated fatty acids.

### 12 Liquid formulation embodiments

The compositions according to the present disclosure typically may be formulated as liquid formulations. The liquid formulation may have a conventional base for a cosmetic composition, for example an aqueous base, an alcoholic base or an aqueous-alcoholic base. The composition in the form of a liquid formulation may comprise (in addition to Avena strigosa extract, Ononis spinosa extract, 2-O-feruloyl-L-malic acid, and optionally Nasturtium officinale extract), for example, 70.0 to 99.5 percent-by-weight water-alcohol-based solvent. In particular, the composition in the form of a liquid formulation may comprise, for example, 90.0 to 99.0 percent-by-weight, for example 92.0 to 98.0 percent-by-weight, based on the weight of the composition, water-alcohol-based solvent.

The composition in the form of a liquid formulation may comprise, for example, water in an amount of at least 10 percent-by-weight, based on the weight of the composition, and alcohol in an amount of at least 20 percent-by-weight, based on the weight of the composition. The solvent may comprise, for example, lower univalent alcohols suitable for cosmetic purposes, such as ethanol, n-propanol and isopropanol, butanol(s), pentanol(s), or multivalent alcohols, such as glycerol, ethylene glycol, propylene glycol, butylene glycol, pentylene glycol, or a combination thereof. The composition in the form of a liquid formulation may further comprise solubilisation enhancers such as for example non-ionic surfactants. The pH of the composition in the form of a liquid formulation may be within the range of from 3.0 to 8.0, in particular within the range of from 4.0 to 6.50, for example within the range of from 4.50 to 5.50. Such composition in the form of a liquid formulation may be a hair tonic.

According to embodiments, the composition in the form of a liquid formulation may comprise a water-based solvent and one or more surfactants, in an amount of 5.0 to 25.0 percent-by-weight, based on the weight of the composition, wherein the surfactant(s) comprise(s) at least one anionic surfactant. Such composition in the form of a liquid formulation may be a shampoo.

According to embodiments, the composition in the form of a liquid formulation may comprise an alcohol-based or water-alcohol-based solvent and one or more surfactants, in an amount of 5.0 to 45.0 percent-by-weight, based on the weight of the composition, wherein the surfactant(s) comprise(s) at least one anionic surfactant. The solvent may comprise, for example, lower univalent alcohols suitable for cosmetic purposes, such as ethanol, n-propanol and isopropanol, butanol(s), pentanol(s), or multivalent alcohols, such as glycerol, ethylene glycol, propylene glycol, butylene glycol, pentylene glycol, or a combination thereof. Such composition in the form of a liquid formulation may be a shampoo.

### 12.1 Scalp-and-hair treatment product

According to embodiments, the compositions according to the present disclosure may be formulated as ready-to-use scalp-and-hair treatment products. For example, the compositions according to the present disclosure may be a ready-to-use scalp-and-hair treatment product selected from hair tonic, hair lotion, hair conditioner, hair cream, hair shampoo, hair rinse, hair liquid, hair oil, hair pomade, hair foam, hair spray. The ready-to-use scalp-and-hair treatment products as well may be used for skin region comprising facial hair, or similar products may be formulated for skin regions comprising facial hair. When referring to "scalp" herein, it is understood that such reference applies equally to part of the scalp region, or a skin region comprising facial hair, or part of such skin region.

According to preferred embodiments, after application the compositions according to the present disclosure are left on the scalp for a time sufficient to trigger the effect of an active ingredient combination according to the present disclosure. For example, the compositions may be left on scalp (or a skin region comprising facial hair) for at least 5 minutes, for example at least 15 minutes. The compositions may be left on scalp for at least 30 minutes, for example at least 60 minutes.

According to preferred embodiments, the ready-to-use scalp-and-hair treatment product is a leave-on product. Leave-on products are applied to hair and/or scalp (or a skin region comprising facial hair), and are not subsequently actively removed.

### 12.2 Hair tonic

According to specific embodiments, the composition according to the present disclosure is a hair tonic, comprising:
0.30 to 3.0 percent-by-weight Avena strigosa extract,
0.30 to 3.0 percent-by-weight Ononis spinosa extract,
0.010-2.0 ppm-by-weight 2-O-feruloyl-L-malic acid,
0.010 to 0.60 percent-by-weight solubilisation enhancer,
0.010 to 0.80 percent-by-weight fragrance,
15.0 to 80.0 percent-by-weight alcohol,
ad 100 percent-by-weight water.

In case the active ingredient is present in the form of Nasturtium officinale extract, the hair tonic according to the present invention may comprise:
0.30 to 3.0 percent-by-weight Avena strigosa extract,
0.30 to 3.0 percent-by-weight Ononis spinosa extract,
0.50 to 5.0 percent-by-weight Nasturtium officinale extract comprising 2-O-feruloyl-L-malic acid,
0.010 to 0.60 percent-by-weight solubilisation enhancer,
0.010 to 0.80 percent-by-weight fragrance,
15.0 to 80.0 percent-by-weight alcohol,
ad 100 percent-by-weight water.

According to embodiments, the solubilisation enhancer of the hair tonic may be selected from PEG-ylated Castor Oil, hydrogenated PEG-ylated Castor Oil, or a combination thereof.

According to embodiments, the alcohol of the hair tonic may be ethanol, or propanol, or a blend of ethanol and propanol. According to particular embodiments, the alcohol of the hair tonic may be a blend of 70-50 parts-by-weight ethanol and/or propanol, and 30-50 parts-by-weight ethylene glycol and/or propylene glycol. According to a typical embodiment, the main component of the solvent system of the hair tonic is aqueous ethanol.

According to embodiments, the hair tonic further may comprise a supportive component. Examples of such supportive components include in particular vasodilatators, blood circulation stimulators, antioxidants, vitamins, provitamins. If present, the amount of the supportive component or combinations thereof will be in the range of from 0.0010 to 5.0, in particular from 0.010 to 3.0 percent-by-weight, based on the weight of the hair tonic.

The supportive component may comprise, for example, one or more of tocopherols, tocotrienols, tocopherol esters, nicotinic acid, nicotinic amides, nicotinic esters, vasodilatory plant extracts, camphor, royal jelly, biotin, vitamin B3, vitamin B12, vitamin C, vitamin D2, vitamin D3, vitamin E, vitamin K1, vitamin K2, panthenol (provitamin B5), omega-3 fatty acids, omega-6 fatty acids, folates, cannabidiol, saw palmetto, minoxidil, or combinations thereof.

According to embodiments, the supportive component includes a vasodilator selected from tocopherols (alpha-, beta-, gamma- or delta-tocopherol), in particular alpha-tocopherol, tocopherol esters (including, for example, tocopherol acetate, tocopheryl succinate, tocopherylnicotinate, tocopherylpoly(oxyethylene)-succinate), nicotinic acid, nicotinic amides, nicotinic esters, camphor; an antioxidant such as vitamin C or polyphenol(s); an inhibitor of testosterone-5-alpha-reductase such as the omega-6 fatty acid arachidonic acid; a substance providing a benefit to hair health such as nicotinic amide (niacinamide); or a combination thereof. According to embodiments, the supportive component includes tocopherol or tocopheryl acetate (vitamin E acetate), and/or niacinamide. Suitable vasodilatory plant extracts include burned or distilled nettle extract (Urtica Diocia), roast chestnut extract (Aesculus Hippocastanum) or Arnica extract (Arnica Montana).

According to specific embodiments, the hair tonic may include a supportive component selected from tocopherols, tocopherol esters, nicotininc acid, nicotinic amides, vasodilatory plant extracts, camphor, royal jelly, vitamin B3, vitamin B12, vitamin C, vitamin D2, vitamin D3, vitamin E, cannabidiol, minoxidil, or combinations thereof.

According to embodiments, the hair tonic further may comprise an oil component. The oil component may comprise, for example, peppermint oil, rosemary essential oil, lavender essential oil, tea tree oil, ylang-ylang, thyme oil, cedarwood oil, clary sage essential oil, bergamot oil, jojoba oil, sunflower oil, hydrogenated ethylhexyl olivate, or a combination thereof. If present, the oil component will be present in the hair tonic in an amount of from 0.010 to 0.50, in particular from 0.025 to 0.25 percent-by-weight, based on the weight of the hair tonic.

According to embodiments, the oil component of the hair tonic further may be selected from peppermint oil, rosemary essential oil, thyme oil, cedarwood oil, bergamot oil, jojoba oil, hydrogenated ethylhexyl olivate, or a combination thereof. For example, the oil component of the hair tonic, if present, may include peppermint oil and/or hydrogenated ethylhexyl olivate.

According to embodiments, the hair tonic further may comprise a mineral component. If present, a preferred mineral component of the hair tonic is, for example, zinc in a concentration of 0.0050-0.50 molar.

According to embodiments, the hair tonic further may comprise one or more cosmetically acceptable additives selected from perfumes, opacifiers, preservatives, pH modifiers, chelators, hair-care materials or skin-care materials, physiologically compatible silicone derivative compounds, light protecting agents, anti-flaking agents, hair luster-imparting agents, combability improving agents, defatting agents, moisturizers, conditioning agents, viscosity modifiers, cooling agents, or combinations thereof. The total amount of these cosmetically acceptable additives may be within a range of from 0.010 to 5.0, conveniently within a range of from 0.010 to 3.0 percent-by-weight, based on the weight of the hair tonic.

Examples and individual amount ranges for these cosmetically acceptable additives are indicated above in Chapter 10. According to embodiments, the hair tonic may comprise a moisturizer and/or a cooling agent. According to embodiments, the moisturizer may be selected from niacinamide, sodium PCA, glycine. According to embodiments, the cooling agent may be selected from menthol, menthyl lactate, isopulegol.

According to embodiments, the hair tonic may be essentially free of added compounds selected from quaternary ammonium compounds, silicones, or a combination thereof. According to particular embodiments, the hair tonic may be free of added compounds selected from quaternary ammonium compounds, silicones, or a combination thereof.

According to embodiments, the hair tonic may be essentially free of added compounds selected from biotin, unsaturated fatty acids, non-film-forming polymers, or a combination thereof. In particluar embodiments, the hair tonic may be free of added biotin, unsaturated fatty acids, non-film-forming polymers, or a combination thereof.

For example, the hair tonic may be essentially free or be free of added compounds selected from quaternary ammonium compounds, silicones, biotin, unsaturated fatty acids, non-film-forming polymers, or a combination thereof.

According to embodiments, the hair tonic may comprise:
1.50 to 3.50 percent-by-weight niacinamide (nicotinic amide),
0.10 to 0.50 percent-by-weight PEG-40 Hydrogenated Castor Oil,
20.0 to 40.0 percent-by-weight ethanol.

According to embodiments, the hair tonic may:
0.10 to 2.0 percent-by-weight Sandal Pentanol,
0.10 to 2.0 percent-by-weight caffeine,
0.010 to 3. 0 percent-by-weight of a C10-C18 fatty acid or an alkai metal salt thereof,
or a combination thereof.

According to specific embodiments, the hair tonic according to the present invention may comprise:
0.30 to 3.0 percent-by-weight Avena strigosa extract,
0.30 to 3.0 percent-by-weight Ononis spinosa extract,
0.50 to 5.0 percent-by-weight Nasturtium officinale extract comprising 2-O-feruloyl-L-malic acid,
0.10 to 0.50 percent-by-weight PEG-40 Hydrogenated Castor Oil,
2.0 to 3.50 percent-by-weight glycerol,
0.010 to 0.80 percent-by-weight fragrance,
20.0 to 40.0 percent-by-weight ethanol,
optionally, 0.10 to 2.0 percent-by-weight caffeine,
optionally, 1.50 to 3.50 percent-by-weight niacinamide,
optionally, 0.10 to 1.0 percent-by-weight xanthan gum
ad 100 percent-by-weight water.

According to even more specific embodiments, the hair tonic according to the present invention may essentially consist of:
0.60 to 1.80 percent-by-weight Avena strigosa extract,
0.60 to 1.80 percent-by-weight Ononis spinosa extract,
1.50 to 3.0 percent-by-weight Nasturtium officinale extract comprising 2-O-feruloyl-L-malic acid,
0.10 to 0.50 percent-by-weight PEG-40 Hydrogenated Castor Oil,
2.0 to 3.50 percent-by-weight glycerol,
0.20 to 0.80 percent-by-weight fragrance,
25.0 to 35.0 percent-by-weight ethanol,
optionally, 0.10 to 0.50 percent-by-weight caffeine,
optionally, 2.20 to 2.80 percent-by-weight niacinamide,
optionally, 0.20 to 0.60 percent-by-weight xanthan gum
ad 100 percent-by-weight water.

### 13 Methods and uses

The compositions or hair tonics according to the present disclosure may be employed according to a method wherein the composition or hair tonic is applied to at least part of a scalp region, or to at least part of a skin region comprising facial hair. Conveniently, the composition or hair tonic may be applied to (at least part of) the scalp (or to at least part of the skin region comprising facial hair) and subsequently or simultaneously be gently worked in.

The compositions or hair tonics according to the present disclosure are applied in an amount sufficient to obtain the desired effect. In particular, the compositions or hair tonics may be applied applied to the scalp in order to deliver an effective amount of the combination of active ingredients. For example, the compositions or hair tonics may be applied applied to the scalp at a daily dose of 0.50 to 20.0 ml. According to a particular example, the compositions or hair tonics may be applied applied to the scalp at a daily dose of 1.50 to 10.0 ml.

The compositions or hair tonics according to the present disclosure may be applied applied to the scalp in one, two or three applications per day. For example, the compositions or hair tonics may be applied applied to the scalp in one or two applications per day.

The compositions or hair tonics according to the present disclosure are left on a scalp region or skin region comprising facial hair for sufficient time to obtain the desired effect. In particular, the compositions or hair tonics may be leave-on products, which after application to hair and/or scalp are not - or at least not immediately - rinsed or washed out.

According to particular embodiments of the method according to the present invention, the method is for reducing or preventing the greying of hair. According to even more particular embodiments, the method is for reverting the color of grey hair. As used herein, the term "reverting the color of grey hair" typically is associated with a positive effect on melanin re-pigmentation, which in consequence leads to a hair color of a given individual perceived less greyish or whitish, and at least in part resembling closer to the original hair color of the individual prior to turning grey.

Further according to even more particular embodiments, the method according to the present invention is for improving scalp health.

Further according to even more particular embodiments, the method according to the present invention is for improving the structure of hair affected by age-related hair thinning, for reducing hair loss, or both.

As far as the present invention relates to the above methods, it is considered that the method aspects according to the present invention relate to cosmetic methods.

Further subject matter of the present disclosure is the use of the compositions or hair tonics according to the present invention for reducing or preventing the greying of hair and improving scalp health. A particular aspect of the present invention concerns using the compositions or hair tonics according to the present invention for reverting the color of grey hair.

Further subject matter of the present disclosure is the use of the compositions or hair tonics according to the present invention for promoting natural mammalian hair growth and/or reducing mammalian hair loss.

### 14 EXAMPLES

The following examples should illustrate the invention claimed below in greater detail, but the details of these examples should not be considered as limiting the claims appended hereinbelow.

### 14.1 Example 1 Consumer test study

The effect of a composition according to the present invention was investigated in a consumer study over a period of four months. 25 female and male test persons aged between 18 and 45 years, and with 10% to 30% grey hair, participated in the study. 24 test persons finished the study.

### 14.1.1 Test composition

| component | component type | amount [percent-by-weight] |
|---|---|---|
| Agreynist^{®} | active ingredient(s) | 2.500 |
| Burgeon-Up | active ingredient(s) | 2.000 |
| Glycerin 86.0 % in water | moisturizer | 3.000 |
| xanthan gum | viscosity modifier | 0.400 |
| PEG-40 Hydrogenated Castor Oil, 10 % Wa | surfactant | 0.200 |
| Mosa Mint Oil | skin conditioner | 0.040 |
| citric acid anhydrous | pH adjuster | 0.025 |
| Ethanol | solvent | 30.000 |
| Water Purified | solvent | 61.835 |
| total | | 100.000 |

Three pipettes of the test composition were applied. The application was demonstrated by a technician during the first application on day 1. The test composition was then applied on the scalp once daily in the evening, for 16 weeks without rinsing off by the test persons at home and gently massaged according to application training. If hair washing was required, the test composition was applied afterwards.

The effect of the test composition was assessed by images of the head (HiRIS, High Resolution Imaging System) at the beginning, after one month and four months of product application. The images were subsequently ranked by trained graders regarding grey hair. Additionally, images by DermLite were taken after one month and four months of product application. At the end of the study, one strand of approximately 150 hairs was removed for counting the hairs, the color of which had changed from grey to pigmented. Further, at the end of the study, the test persons filled in a questionnaire.

### 14.1.2 Study results

The grey appearance of the hair of the test persons significantly decreased by 14% after 16 weeks of product application in comparison to baseline according to the trained grader assessments. No significant difference was found after four weeks compared to baseline, or between Week 4 and Week 16.

AFTER 16 WEEKS OF TREATMENT WITH THE TEST COMPOSITION, THE GREY APPEARANCE OF HAIR IMPROVED SIGNIFICANTLY COMPARED TO PRIOR PRODUCT APPLICATION.

### 14.1.3 Questionnaire

Product traits were assessed by the test persons regarding the following topics:

| | | |
|---|---|---|
| Q1 | improvement of scalp health | 100% |
| Q2 | fuller look of the hair | 63.6% |
| Q3 | thicker feel of the hair | 54.5% |
| Q4 | healthier look of the hair | 72.7% |
| Q5 | more shine of the hair | 50.0% |

As regards the question concerning the effect of the test composition on scalp health, the clear majority of the test persons (100%) agreed that the test product had a positive effect on scalp health, or generally improved scalp health. A high level of agreement was found for an improved appearance in terms of a "healthier look of the hair" or a " fuller look of the hair". About half of the test persons confirmed an improved appearance in terms of "thicker feel of the hair" or "more shine of the hair".

A NOTABLE EFFECT OF THE TEST COMPOSITION ON THE IMPROVEMENT OF SCALP HEALTH, AND EFFECTS OF THE TEST COMPOSITION TO MAKE THE HAIR LOOK "HEALTHIER" OR "FULLER" WERE ASSESSED POSITIVELY BY THE TEST PERSONS.

### 15 STATEMENTS

The following statements further illustrate the present invention.
1 Composition, comprising an Avena strigosa extract, an Ononis spinosa extract, and 2-O-feruloyl-L-malic acid.
2 The composition according to statement 1, wherein the Avena strigosa extract is water-based, alcohol-based or water-alcohol-based, and the Ononis spinosa extract is water-based, alcohol-based or water-alcohol-based.
3 The composition according to statement 1 or 2, wherein the Avena strigosa extract comprises water and an alcohol selected from ethanol, propanol, butanol, pentanol, hexanol, glycerol, ethylene glycol, propylene glycol, butylene glycol, pentylene glycol or a combination thereof, and the Ononis spinosa extract comprises water and an alcohol selected from ethanol, propanol, butanol, pentanol, hexanol, glycerol, ethylene glycol, propylene glycol, butylene glycol, pentylene glycol, or a combination thereof.
4 The composition according to any of the preceding statements, wherein the Avena strigosa extract is a hydrolyzate, or the Ononis spinosa extract is a hydrolyzate, or both are a hydrolyzate.
5 The composition according to any of the preceding statements, wherein the Avena strigosa extract is an aqueous glycolic extract, or the Ononis spinosa extract is an aqueous glycolic extract, or both are an aqueous glycolic extract.
6 The composition according to any of the preceding statements, wherein the Avena strigosa extract has a solids content in the range of 0.10-16.0 percent-by-weight, in particular a solids content in the range of 0.2-10.0 percent-by-weight, such as a solids content in the range of 0.50-8.0 percent-by-weight, and the Ononis spinosa extract has a solids content in the range of 0.10-16.0 percent-by-weight, in particular a solids content in the range of 0.2-10.0 percent-by-weight, such as a solids content in the range of 0.50-8.0 percent-by-weight.
7 The composition according to any of the preceding statements, wherein the Avena strigosa extract comprises peptides having a molecular mass of less than 2.0 kDa, in particular in an amount of 15.0-70.0 percent-by-weight, based on the extract's solids content, or the Ononis spinosa extract comprises peptides having a molecular mass of less than 2.0 kDa, in particular in an amount of 15.0-70.0 percent-by-weight, based on the extract's solids content, or both comprise peptides having a molecular mass of less than 2.0 kDa, in particular in an amount of 15.0-70.0 percent-by-weight, based on the extract's solids content.
8 The composition according to any of the preceding statements, wherein the Avena strigosa extract comprises flavenoids, phenolic acids, and/or polyphenolic compounds, in particular in an amount of 15.0-70.0 percent-by-weight, based on the extract's solids content, or the Ononis spinosa extract comprises flavenoids, phenolic acids, and/or polyphenolic compounds, in particular in an amount of 15.0-70.0 percent-by-weight, based on the extract's solids content, or both comprise flavenoids, phenolic acids, and/or polyphenolic compounds, in particular in an amount of 15.0-70.0 percent-by-weight, based on the extract's solids content.
9 The composition according to any of the preceding statements, comprising 2-O-feruloyl-L-malic acid in a concentration of 0.0010-20.0 ppm-by-weight, in particular 0.010-2.0 ppm-by-weight, such as 0.050-0.20 ppm-by-weight, based on the total weight of the composition.
10 The composition according to any of the preceding statements, comprising Nasturtium officinale extract.
11 The composition according to statement 10, wherein the Nasturtium officinale extract comprises water and an alcohol selected from ethanol, propanol, butanol, pentanol, hexanol, glycerol, ethylene glycol, propylene glycol, butylene glycol, pentylene glycol, or a combination thereof.
12 The composition according to statement 10 or 11, wherein the Nasturtium officinale extract is an aqueous ethanol extract.
13 The composition according to any of statements 10 to 12, wherein the Nasturtium officinale extract has a solids content in the range of 0.10-16.0 percent-by-weight, in particular a solids content in the range of 0.20-10.0 percent-by-weight, such as a solids content in the range of 0.50-8.0 percent-by-weight.
14 The composition according to any of statements 10 to 13, wherein the Nasturtium officinale extract comprises peptides having a molecular mass of less than 2.0 kDa, in particular in an amount of 15.0-70.0 percent-by-weight, based on the Nasturtium officinale extract's solids content.
15 The composition according to any of statements 10 to 14, wherein the Nasturtium officinale extract comprises flavenoids, phenolic acids, and/or polyphenolic compounds, in particular in an amount of 15.0-70.0 percent-by-weight, based on the Nasturtium officinale extract's solids content.
16 The composition according to any of the preceding statements, comprising 0.20-5.0 percent-by-weight, based on the total weight of the composition, Avena strigosa extract, and 0.20-5.0 percent-by-weight, based on the total weight of the composition, Ononis spinosa extract.
17 The composition according to statement 16, comprising 0.30 to 3.0 percent-by-weight, based on the total weight of the composition, Avena strigosa extract, and 0.30 to 3.0 percent-by-weight, based on the total weight of the composition, Ononis spinosa extract.
18 The composition according to any of the preceding statements, comprising 50.0 to 2000.0 ppm-by-weight, based on the extract's solids content, Avena strigosa extract, and 50.0 to 2000.0 ppm-by-weight, based on the extract's solids content, Ononis spinosa extract.
19 The composition according to statement 18, comprising 100.0 to 800.0 ppm-by-weight, based on the extract's solids content, Avena strigosa extract, and 100.0 to 800.0 ppm-by-weight, based on the extract's solids content, Ononis spinosa extract.
20 The composition according to any of the preceding statements, comprising 0.40-10.0 percent-by-weight, based on the total weight of the composition, Nasturtium officinale extract, in particular 0.80-5.0 percent-by-weight, based on the total weight of the composition, Nasturtium officinale extract.
21 The composition according to any of the preceding statements, comprising 4.0 to 400.0 ppm-by-weight, based on the extract's solids content, Nasturtium officinale extract, in particular 16.0 to 100.0 ppm-by-weight, based on the extract's solids content, Nasturtium officinale extract.
22 The composition according to any of the preceding statements, wherein a weight ratio of Avena strigosa extract to Ononis spinosa extract is in the range of 0.20-5.0, in particular in the range of 0.50-2.0, such as in the range of 0.80-1.20.
23 The composition according any of the preceding statements, comprising Nasturtium officinale extract, wherein a weight ratio of Avena strigosa extract to Ononis spinosa extract to Nasturtium officinale extract is in the range of [0.20-5.0] : [0.20-5.0] : [0.40-8.0].
24 The composition according to statement 23, wherein a weight ratio of Avena strigosa extract to Ononis spinosa extract to Nasturtium officinale extract is in the range of [0.60-2.0] : [0.60-2.0] : [0.80-4.0].
25 The composition according to statement 24, wherein a weight ratio of Avena strigosa extract to Ononis spinosa extract to Nasturtium officinale extract is in the range of [0.80-1.20] : [0.80-1.20] : [1.0-2.5].
26 The composition according to any of the preceding statements, further comprising sandal pentanol (3-methyl-5-(2,2,3-trimethylcyclopent-3-en-1-yl)pentan-2-ol).
27 The composition according to statement 26, wherein the sandal pentanol is present in an amount of from 0.010 to 10.0 percent-by-weight, in particular in an amount of 0.10 to 2.0 percent-by-weight, based on the weight of the composition.
28 The composition according to any of the preceding statements, further comprising caffeine, one or more saturated and/or unsaturated C10-C18 fatty acids, or a combination thereof.
29 The composition according to statement 28, wherein the C10-C18 fatty acid(s) is/are selected from capric acid (C10:0), lauric acid (C12:0), myristic acid (C14:0), isomyristic acid (C14:0), palmitic acid (C16:0), palmitoleic acid (C16:1), stearic acid (C18:0), oleic acid (C18:1), linoleic acid (C18:2), linolenic acid (C18:3), parinaric acid (C18:4), petroselinic acid (C18:1), or combinations thereof.
30 The composition according to statements 28 or 29, wherein the C10-C18 fatty acid(s) is/are present in the form of free acid, alkali metal salt, alkaline earth metal salt, ammonium salt or combination thereof, in particular wherein the fatty acid(s) is/are present in the form of free acid(s).
31 The composition according to any of statements 28 to 30, wherein the one or more C10-C18 fatty acid(s) is/are present in an amount of from 0.010 to 10.0 percent-by-weight, in particular in an amount of 0.10 to 2.0 percent-by-weight, based on the weight of the composition.
32 The composition according to any of statements 28 to 31, wherein the caffeine is present in an amount of from 0.010 to 10.0 percent-by-weight, in particular in an amount of 0.10 to 2.0 percent-by-weight, based on the weight of the composition.
33 The composition according to any of the preceding statements, further comprising a supportive component selected from vasodilatators, blood circulation stimulators, antioxidants, vitamins, provitamins, and combinations thereof.
34 The composition according to statement 33, wherein the supportive component is present in an amount of from 0.0010 to 5.0, in particular from 0.010 to 3.0 percent-by-weight, based on the weight of the composition.
35 The composition according to statement 33 or 34, wherein the supportive component comprises one or more of tocopherols, tocotrienols, tocopherol esters, nicotinic acid, nicotinic amides, nicotinic esters, vasodilatory plant extracts, camphor, royal jelly, biotin, vitamin B3, vitamin B12, vitamin C, vitamin D2, vitamin D3, vitamin E, vitamin K1, vitamin K2, panthenol, omega-3 fatty acids, omega-6 fatty acids, folates, cannabidiol, , amino acids or combinations thereof.
36 The composition according to any of the preceding statements, further comprising an oil component selected from peppermint oil, rosemary essential oil, lavender essential oil, tea tree oil, ylang-ylang, thyme oil, cedarwood oil, clary sage essential oil, bergamot oil, jojoba oil, sunflower oil, hydrogenated ethylhexyl olivate, or a combination thereof.
37 The composition according to statement 36, wherein the oil component is present in an amount of from 0.0010 to 1.0, in particular from 0.010 to 0.50 percent-by-weight, based on the weight of the composition.
38 The composition according to any of the preceding statements, further comprising a mineral component selected from iron, zinc, or a combination thereof.
39 The composition according to statement 38, wherein the mineral component is present in 0.0050-0.50 molar concentration.
40 The composition according to any of the preceding statements, further comprising one or more cosmetically acceptable additives selected from perfumes, opacifiers, preservatives, pH modifiers, chelators, hair-care materials or skin-care materials, physiologically compatible silicone derivative compounds, light protecting agents, anti-flaking agents, hair luster-imparting agents, combability improving agents, defatting agents, moisturizers, conditioning agents, viscosity modifiers, cooling agents, or combinations thereof.
41 The composition according to any of the preceding statements, further comprising a solvent.
42 The composition according to statement 41, wherein the solvent is water-based, alcohol-based or water-alcohol-based.
43 The composition according to statement 41 or 42, wherein the solvent comprises water and an alcohol selected from ethanol, propanol, butanol, pentanol, hexanol, glycerol, ethylene glycol, propylene glycol, butylene glycol, pentylene glycol or a combination thereof.
44 The composition according to any of statements 41 to 43, further comprising a solubilisation enhancer.
45 The composition according to statement 44, wherein the solubilisation enhancer comprises one or more surfactants.
46 The composition according to any of the preceding statements, having a pH within the range of from 3.0 to 8.0, in particular within the range of from 4.0 to 6.50, such as within the range of from 4.50 to 5.50.
47 The composition according to any of the preceding statements, having a viscosity within the range of 0.50 mPa•s to 10.0 Pa•s.
48 The composition according to any of the preceding statements, wherein the composition is essentially free of silicones.
49 The composition according to any of the preceding statements, wherein the composition is essentially free of non-film-forming polymers.
50 The composition according to any of the preceding statements, wherein the composition is a ready-to-use scalp-and-hair treatment product selected from hair tonic, hair lotion, hair conditioner, hair cream, hair shampoo, hair rinse, hair liquid, hair oil, hair pomade, hair foam, hair spray.
51 The composition according to statement 50, wherein the composition is a leave-on product.
52 A hair tonic, comprising:
   0.30 to 3.0 percent-by-weight Avena strigosa extract,
   0.30 to 3.0 percent-by-weight Ononis spinosa extract,
   0.010-2.0 ppm-by-weight 2-O-feruloyl-L-malic acid,
   0.010 to 0.60 percent-by-weight solubilisation enhancer,
   0.010 to 0.80 percent-by-weight fragrance,
   15.0 to 80.0 percent-by-weight alcohol,
   ad 100 percent-by-weight water.
53 A hair tonic, comprising:
   0.30 to 3.0 percent-by-weight Avena strigosa extract,
   0.30 to 3.0 percent-by-weight Ononis spinosa extract,
   0.50 to 5.0 percent-by-weight Nasturtium officinale extract comprising 2-O-feruloyl-L-malic acid,
   0.010 to 0.60 percent-by-weight solubilisation enhancer,
   0.010 to 0.80 percent-by-weight fragrance,
   15.0 to 80.0 percent-by-weight alcohol,
   ad 100 percent-by-weight water.
54 The hair tonic according to statements 52 or 53, further comprising 0.050 to 3.50 percent-by-weight of a supportive component comprising one or more of tocopherols, tocotrienols, tocopherol esters, nicotinic acid, nicotinic amides, nicotinic esters, vasodilatory plant extracts, camphor, royal jelly, biotin, vitamin B3, vitamin B12, vitamin C, vitamin D2, vitamin D3, vitamin E, vitamin K1, vitamin K2, panthenol, omega-3 fatty acids, omega-6 fatty acids, folates, cannabidiol, saw palmetto, minoxidil, or combinations thereof.
55 The hair tonic according to statement 54, wherein the supportive component is selected from tocopherols, tocopherol esters, nicotininc acid, nicotinic amides, vasodilatory plant extracts, camphor, royal jelly, vitamin B3, vitamin B12, vitamin C, vitamin D2, vitamin D3, vitamin E, cannabidiol, minoxidil, or combinations thereof.
56 The hair tonic according to any of statements 52 to 55, further comprising 0.025 to 0.25 percent-by-weight of an an oil component selected from peppermint oil, rosemary essential oil, lavender essential oil, tea tree oil, ylang-ylang, thyme oil, cedarwood oil, clary sage essential oil, bergamot oil, jojoba oil, sunflower oil, hydrogenated ethylhexyl olivate, or a combination thereof.
57 The hair tonic according to statement 56, wherein the oil component is selected from peppermint oil, rosemary essential oil, thyme oil, cedarwood oil, bergamot oil, jojoba oil, hydrogenated ethylhexyl olivate, or a combination thereof.
58 The hair tonic according to any of statements 52 to 57, further comprising zinc in a concentration of 0.0050-0.50 molar.
59 The hair tonic according to any of statements 52 to 58, further comprising 0.010 to 5.0 percent-by-weight of one or more cosmetically acceptable additives selected from perfumes, opacifiers, preservatives, pH modifiers, chelators, hair-care materials or skin-care materials, physiologically compatible silicone derivative compounds, light protecting agents, anti-flaking agents, hair luster-imparting agents, combability improving agents, defatting agents, moisturizers, conditioning agents, viscosity modifiers, cooling agents, or combinations thereof.
60 The hair tonic according to any of statements 52 to 59, comprising a moisturizer and/or a cooling agent.
61 The hair tonic according to any of statements 52 to 60, comprising:
   1.50 to 3.50 percent-by-weight niacinamide (nicotinic amide)
   0.10 to 0.50 percent-by-weight PEG-40 Hydrogenated Castor Oil,
   20.0 to 40.0 percent-by-weight ethanol.
62 The hair tonic according to any of statements 52 to 61, further comprising:
   0.10 to 2.0 percent-by-weight Sandal Pentanol,
   0.10 to 2.0 percent-by-weight caffeine,
   0.010 to 3. 0 percent-by-weight of a C10-C18 fatty acid or an alkai metal salt thereof,
   or a combination thereof.
63 A method of treating hair, wherein the method comprises applying the composition according to any of statements 1 to 51 or the hair tonic according to any of statements 52-62 to at least part of a scalp region, or to at least part of a skin region comprising facial hair.
64 The method according to statement 63, comprising applying the composition or hair tonic at a daily dose of 0.50 to 20.0 ml, in particular at a daily dose of 1.50 to 10.0 ml.
65 The method according to statement 63 or 64, comprising applying the composition in one, two or three applications per day.
66 The method according to any of statements 63 to 65, for reducing or preventing the greying of hair.
67 The method according to any of statements 63 to 66, for reverting the color of grey hair.
68 The method according to any of statements 63 to 67, for improving scalp health.
69 The method according to any of statements 63 to 68, for improving the structure of hair affected by age-related hair thinning.
70 The method according to any of statements 63 to 69, for reducing hair loss.
71 The method according to any of statements 63 to 70, wherein the method is a cosmetic method.
72 The composition according to any of statements 1 to 51 or the hair tonic according to any of statements 52-62, for use in a method of reducing or preventing the greying of hair and improving scalp health.
73 The composition or hair tonic for use according to statement 72, for use in a method of reverting the color of grey hair.
74 Use of the composition according to any of statements 1 to 51 or the hair tonic according to any of statements 52-62 for reducing or preventing the greying of hair and improving scalp health.
75 The use according to statement 67 for reverting the color of grey hair.

## Claims

1. Composition, comprising an Avena strigosa extract, an Ononis spinosa extract, and 2-O-feruloyl-L-malic acid.

2. The composition according to claim 1, wherein the Avena strigosa extract comprises water and an alcohol selected from ethanol, propanol, butanol, pentanol, hexanol, glycerol, ethylene glycol, propylene glycol, butylene glycol, pentylene glycol or a combination thereof, and the Ononis spinosa extract comprises water and an alcohol selected from ethanol, propanol, butanol, pentanol, hexanol, glycerol, ethylene glycol, propylene glycol, butylene glycol, pentylene glycol, or a combination thereof.

3. The composition according to claim 1 or 2, comprising 2-O-feruloyl-L-malic acid in a concentration of 0.0010-20.0 ppm-by-weight, in particular 0.010-2.0 ppm-by-weight, such as 0.050-0.20 ppm-by-weight, based on the total weight of the composition.

4. The composition according to any of the preceding claims, comprising Nasturtium officinale extract, in particular wherein the Nasturtium officinale extract comprises water and an alcohol selected from ethanol, propanol, butanol, pentanol, hexanol, glycerol, ethylene glycol, propylene glycol, butylene glycol, pentylene glycol, or a combination thereof.

5. The composition according to any of the preceding claims, comprising 0.20-5.0 percent-by-weight, based on the total weight of the composition, Avena strigosa extract, and 0.20-5.0 percent-by-weight, based on the total weight of the composition, Ononis spinosa extract, for example comprising 0.30 to 3.0 percent-by-weight, based on the total weight of the composition, Avena strigosa extract, and 0.30 to 3.0 percent-by-weight, based on the total weight of the composition, Ononis spinosa extract

6. The composition according to any of the preceding claims, comprising 0.40-10.0 percent-by-weight, based on the total weight of the composition, Nasturtium officinale extract, in particular 0.80-5.0 percent-by-weight, based on the total weight of the composition, Nasturtium officinale extract.

7. The composition according to any of the preceding claims, wherein a weight ratio of Avena strigosa extract to Ononis spinosa extract is in the range of 0.20-5.0, in particular in the range of 0.50-2.0, such as in the range of 0.80-1.20.

8. The composition according to any of the preceding claims, comprising Nasturtium officinale extract, wherein a weight ratio of Avena strigosa extract to Ononis spinosa extract to Nasturtium officinale extract is in the range of [0.20-5.0] : [0.20-5.0] : [0.40-8.0], in particular wherein a weight ratio of Avena strigosa extract to Ononis spinosa extract to Nasturtium officinale extract is in the range of [0.60-2.0] : [0.60-2.0] : [0.80-4.0], for example wherein a weight ratio of Avena strigosa extract to Ononis spinosa extract to Nasturtium officinale extract is in the range of [0.80-1.20] : [0.80-1.20] : [1.0-2.5].

9. The composition according to any of the preceding claims, further comprising sandal pentanol (3-methyl-5-(2,2,3-trimethylcyclopent-3-en-1-yl)pentan-2-ol), caffeine, one or more saturated and/or unsaturated C10-C18 fatty acids, or a combination thereof

10. The composition according to any of the preceding claims, further comprising a solvent, in particular wherein the solvent comprises water and an alcohol selected from ethanol, propanol, butanol, pentanol, hexanol, glycerol, ethylene glycol, propylene glycol, butylene glycol, pentylene glycol or a combination thereof, the composition optionally further comprising one or more surfactants.

11. A hair tonic, comprising:
0.30 to 3.0 percent-by-weight Avena strigosa extract,
0.30 to 3.0 percent-by-weight Ononis spinosa extract,
0.010-2.0 ppm-by-weight 2-O-feruloyl-L-malic acid,
0.010 to 0.60 percent-by-weight solubilisation enhancer,
0.010 to 0.80 percent-by-weight fragrance,
15.0 to 80.0 percent-by-weight alcohol,
ad 100 percent-by-weight water.

12. A hair tonic, comprising:
0.30 to 3.0 percent-by-weight Avena strigosa extract,
0.30 to 3.0 percent-by-weight Ononis spinosa extract,
0.50 to 5.0 percent-by-weight Nasturtium officinale extract comprising 2-O-feruloyl-L-malic acid,
0.010 to 0.60 percent-by-weight solubilisation enhancer,
0.010 to 0.80 percent-by-weight fragrance,
15.0 to 80.0 percent-by-weight alcohol,
ad 100 percent-by-weight water.

13. The hair tonic according to any of statements 52 to 60, comprising:
1.50 to 3.50 percent-by-weight niacinamide (nicotinic amide)
0.10 to 0.50 percent-by-weight PEG-40 Hydrogenated Castor Oil,
20.0 to 40.0 percent-by-weight ethanol.

14. A method of treating hair, wherein the method comprises applying the composition according to any of claims 1 to 10 or the hair tonic according to any of claims 11-13 to at least part of a scalp region, or to at least part of a skin region comprising facial hair.

15. Use of the composition according to any of claims 1 to 10 or the hair tonic according to any of claims 11-13 for reducing or preventing the greying of hair and improving scalp health.
